# EUROPEAN PATENT APPLICATION

(11) **EP 1 491 154 A1**
(43) Date of publication of application: **29.12.2004**
(21) Application number: 03707879.7
(22) Date of filing: 22.01.2003
(51) Int. Cl.: A61B 17/30

(54) **CERAMIC-COATED INSTRUMENTS FOR MEDICAL USE, CERAMIC-COATED INSTRUMENTS FOR STUDYING LIVING ORGANISMS AND PROCESS FOR PRODUCING THE SAME**

(30) Priority: 22.01.2002 JP 2002012863; 22.02.2002 JP 2002045894; 06.03.2002 JP 2002060005; 15.04.2002 JP 2002111899; 16.04.2002 JP 2002112764; 28.06.2002 JP 2002190075; 01.10.2002 JP 2002288654; 30.10.2002 JP 2002315474
(71) Applicant: JFE Steel Corporation, Tokyo (JP)
(72) Inventor: INOKUTI, Yukio, c/o Technical Res. Laboratories, Chiba-shi, Chiba 260-0835 (JP); MORI, H., c/o Osaka University, Ibaraki, Osaka 567-0047 (JP); FUKUDA, Hiroyuki, c/o Chiba University, Chiba-shi, Chiba 260-8677 (JP); EBARA, Masaaki, c/o Chiba University, Chiba-shi, Chiba 260-8677 (JP)
(74) Representative: Grünecker, Kinkeldey, Stockmair & Schwanhäusser Anwaltssozietät
(86) International application number: PCT/JP2003/000498
(87) International publication number: WO 2003/061490

(57) **Abstract**

Providing a ceramic-coated medical appliance quite excellent in insulatingperformancewhichhas no negative effects on tissues or cells of an organism subjected to surgery; a ceramic-coated genetic control needle which has no risk of breaking during use nor no unfavorable effects, such as destruction or tear-off, on sliced tissues or tissue areas around the needle at site of penetration into a lesion and which cuts off a cell nucleus or injects an immunological solution into the nucleus without causing any negative effects on tissues around the penetrated needle; and a fabrication method for the same.

Specifically, in the ceramic-coated medial needle, genetic control needle, biopsy forceps and medical appliances such as forceps, surgical scissors and surgical knife, an insulating ceramic film having a resistance ρ of 10⁵ Ω·m or more is formed, by dry plating, at least on a portion, a part or the whole of an outer and/or an inner surface of a metal needle that directly contact an organism, or cells or cell nuclei thereof.

## Description

### Technical Field

The present invention relates to a ceramic-coatedmedical or biopsy appliance formed of metal and a fabrication method therefor. Particularly, the invention relates to medical appliances and biopsy appliances which are coated with ceramic coatings on metallic parts thereof that directly contact any part of an organism, and to a fabrication method for the same. The medical and biopsy appliances principally include medical needles such as injection needles, puncture needles for hepatobiopsy/renal biopsy, suture needles and the like; biopsy forceps; forceps; surgical scissors; surgical knives and the like. The invention further relates to a ceramic-coated genetic control needle and a fabrication method therefor.

### Background Art

The recent medical technologies have made a dramatic leap forward. In liver and pancreas examinations, for example, a variety of examination methods are commonly used for acquiring data not available from blood test. Such examination methods include ultrasonic diagnostics using ultrasound waves; CT (Computed Tomography) ; MRI (Magnetic Resonance Imaging) for producing sectional images of various internal organs based on strong magnetism and electric waves; angiography for visualizing a sate of blood vessels following the injection of a radiopaque contrast medium via a fine catherter, and the like.

Although the presence of a seat of disease such as cancer may be determined by the blood test or the diagnostic imaging method, histopathological examination of the lesion, such as hepatobiopsy, is required for definite diagnosis.

In such biopsies, tissues are normally collected by' directly penetrating a special puncture needle into the lesion.

As to the use of the conventional puncture needle, however, unfavorable effects, such as cellular or tissular destruction and tissue tear-off, on tissue slices collected from the lesion or on tissue areas around the puncture needle at the site of penetration into the lesionhave been pointed out, because a substrate of the needle is formed of a conductive metal, such as stainless steel, high tension steel or the like, having good electrical properties (resistance ρ: 10⁻⁶ - 10⁻⁸ Ω·m). These negative effects are attributed to 1) redo reaction, 2) hydrolysis and 3) binding to antibodies such as Haptein (refer to, for example, S.G.Steinemann: Periodontology 2000, 17 (1998), p7-21).

In contrast, a puncture needle formed of ceramics (hereinafter, simply referred to as "ceramic puncture needle" or "ceramic needle") may be used without exerting unfavorable effects on the collected tissues or the cells around the puncture needle penetrated into the lesion. However, the ceramic puncture needles and the ceramic needles are very fragile and tend to break easily, and therefore are not currently adopted in any case.

The present inventors first contemplated to form a ceramic film over an outer surface of the ordinary metal puncture needle in order to obtain a puncture needle having no negative effects on the collected tissues or the cells around the puncture needle penetrated into the lesion.

If, however, the ceramic film coated on a very fine cylindrical body like the puncture needle has poor adhesion to a surface of the cylindrical body, the ceramic film will be peeled off during use so that the intended object cannot be achieved. Particularly, the puncture needles and injection needles are unavoidably flexed to some degree when used, thus involving a high risk of peeling of the ceramic film.

For instance, Japanese examined patent No.JP-B-6-20464 has disclosed a medical incision/impression device wherein a medical knife is provided with a diamond film coating on its surface for reducing frictional resistance at incision.

However, the diamond film is coated by heating a substrate to 500 to 1300°C, while a thermal expansion difference between the substrate and the diamond film is significant. Hence, the diamond film is prone to delaminate and is not applicable to the puncture needle and the like as a subject matter of the invention.

Recently, the inventors have discovered a new fact that when a thin TiN ceramic filmplasma-coatedon a ferritic stainless steel sheet is subjected to plastic work of 180° bending, the TiN ceramic film exhibits local elongation at cracked portions assuming unique concave shape just as seen in metals [Inokuti ; Yukio: prize-winning photograph at 2001 International Metallographic Contest (Indianapolis, USA, 2001, 11/5-8. held jointly by IMS (International Metallographic Society) and ASM (Amercian Society of Metals)].

The phenomenon suggests that the very fragile ceramic film is also workable, exhibiting metal-like elongation when subjected to plastic work.

Accordingly, the inventors formed the TiN ceramic film on a surface of a stainless steel puncture needle by ceramic coating process in a high-vacuum and high-plasma atmosphere.

It was confirmed that the TiN ceramic film has such an excellent adhesion to the puncture needle that a certain degree of bending does not cause the delamination of the TiN ceramic film.

However, even the puncture needle coated with the TiN ceramic film could not completely eliminate the unfavorable effects, such as cellular or tissular destruction and tissue tear-off, on the collected tissues or on tissue areas around the puncture needle at the site of penetration into the lesion although such effects were not so serious as those caused by the conventional metal puncture needles.

The invention is directed to a solution to the above problem and has an object to provide a ceramic-coated medical appliance or biopsy appliance (hereinafter, simply referred to as "medical appliances" depending upon cases) which may be used without causing any unfavorable effects on the tissues or cells of organisms, the medical appliances including, for example, medical needles such as injection needles, puncture needles, suture needles; biopsy forceps; forceps; surgical scissors; surgical knives and the like.

Another obj ect of the invention is to provide an innovative ceramic-coated medical puncture needle which is free from the risk of breakage during use and which has no unfavorable effects on the collected tissues or the cells around the puncture needle at the site of penetration into the lesion.

Still another object of the invention is to provide a ceramic-coated medical puncture needle which also has the insulating ceramic coating on its inner surface such that the aforementioned negative effects on the tissues may not occur due to contact between the tissues and a metal surface when a large amount of tissues is collected.

In some cases, the ceramic-coated genetic control needle may have unfavorable effects on cell nucleus when used to cut off the cell nucleus or to inject an immunological solution or the like in the nucleus.

That is, the outer surface of the ceramic-coated needle is covered by the insulating ceramic coating, causing no damage to an area around the penetrated ceramic-coated needle. Unfortunately, the coated needle is so fine that the needle is not always have the insulating ceramic coating on its inner surface. Hence, a fear exists that the negative effects may occur because of the contact between the cell nucleus and the metal surface inside of the needle when the nucleus is cut off or injected with the immunological solution or the like.

The invention is directed to the solution to the above problem and has an object to provide a novel ceramic-coated genetic control needle and a fabrication method therefor. The genetic control needle is characterized in that the insulating ceramic film is also formed on the inner surface of the very fine needle, thereby eliminating the unfavorable effects on an area around the needle penetrated into the cell or the like as well as on the nucleus in the needle during the cut-off of cell nuclei or injection of the immunological solution or the like.

The invention is further directed to the improvement of the aforementioned technique and has an object to provide a fabrication method for the ceramic-coated medical appliances. The method is characterized in that a ceramic film of increased adhesion is formed by way of a mixed layer of an iron matrix and the ceramic film produced at an interface therebetween (initial coat layer) and that the ceramic film is further improved in insulation performance and wear resistance by way of an even more increased resistance p at least at its top surface.

### Disclosure of the Invention

The followings are the gist and constitution of the invention.
1. A ceramic-coated medical appliance or biopsy appliance characterized in that at least a metal portion directly contacting an organism is coated with an insulating ceramic film having a resistance ρ of 10⁵ Ω·m or more.
2. A ceramic-coated medical appliance or biopsy appliance according to the above appliance (1) , characterized in that the ceramic film has a thickness of 0.05 to 5.0 µm.
3. A ceramic-coated medical appliance or biopsy appliance according to the above appliance (1) or (2), characterized in that the ceramic film comprises at least one selected from the group consisting of nitrides, carbides or oxides of Al, B, Si, Cr and Ti.
4. A ceramic-coated medical appliance according to any one of the above appliances (1) to (3) , characterized in that the medical appliance is any one of biopsy forceps, forceps, surgical scissors and a surgical knife.
5. A ceramic-coated medical appliance according to any of the above appliances (1) to (3), characterized in that the ceramic-coated medical appliance is any one of an injection needle, puncture needle and suture needle.
6. A ceramic-coated medical needle according to the above appliance (5), characterized in that the needle has the insulating ceramic film of a resistance ρ of 10⁵ Ω·m or more on a part or the whole of a surface thereof.
7. A ceramic-coated medical needle according to the above needle (6), characterized in that the ceramic film has a thickness of 0.05 to 5.0 µm.
8. A ceramic-coated medical needle according to the above needle (6) or (7), characterized in that the ceramic film comprises at least one selected from the group consisting of nitrides, carbides or oxides of Al, B, Si, Cr and Ti.
9. A ceramic-coated medical needle according to any one of the above needles (6), (7) or (8), characterized in that a substrate metal of the coated needle is stainless steel or high tension steel.
10. A ceramic-coatedmedical needle according to the above needle (6), characterized in that a ceramic-coated area of the needle is a part or the whole of an outer surface thereof and a portion of an inner surface thereof that is 1 mm inward from a tip of the needle.
11. A ceramic-coatedmedical needle according to the above needle (10), characterized in that the ceramic film has a thickness of 0.05 to 5.0 µm.
12. A ceramic-coated medical needle according to the above needle (10) or (11), characterized in that the ceramic film comprises at least one selected from the group consisting of nitrides, carbides or oxides of Al, B, Si, Cr and Ti.
13. A ceramic-coated medical needle according to any one of the above needles (10),(11) or (12), characterized in that a substrate metal of the coated needle is stainless steel or high tension steel.
14. A ceramic-coated generic control needle according to any one of the above appliances (1) to (3), characterized in that the ceramic-coated biopsy appliance is a genetic control needle.
15. A ceramic-coated genetic control needle according to the above needle (14), characterized in that a ceramic-coated area of the metal needle is at least a portion of a surface thereof that contacts a cell nucleus in tissue of an organism.
16. A ceramic-coated genetic control needle according to the above needle (14) or (15), characterized in that the coated needle has a diameter of 0.0005 to 0.5 mm.
17. A ceramic-coated genetic control needle according to any one of the above needles (14),(15) or (16), characterized in that the ceramic film comprises at least one selected from the group consisting of nitrides, oxides or carbides of Al, B, Si, Cr and Ti.
18. A ceramic-coated genetic control needle according to the above needle (14) , characterized in that a ceramic-coated area of an outer surface of the metal needle is at least a portion thereof that contacts tissue of an organism, whereas a ceramic-coated area of an inner surface of the metal needle is at least a portion thereof that contacts a cell nucleus in the tissue of the organism.
19. A ceramic-coated genetic control needle according to the above needle (18), characterized in that the coverage of the insulating ceramic film on the inner surface of the metal needle extends 10 µm or more from a tip of the metal needle.
20. A ceramic-coated genetic control needle according to the above needle (18) or (19), characterized in that the ceramic film has a thickness of 0.05 to 5.0 µm.
21. A ceramic-coated genetic control needle according to any one of the above needles (18),(19) or (20), characterized in that the ceramic film comprises at least one selected from the group consisting of nitrides, carbides or oxides of Al, B, Si, Cr and Ti.
22. A ceramic-coated genetic control needle according to any one of the above needles (18) to (21), characterized in that a substrate metal of the needle is stainless steel or high tension steel.
23. A ceramic-coated genetic control needle according to any one of the above needles (18) to (22), characterized in that the needle has a diameter φ of 0.0005 to 0.5 mm.
24. A ceramic-coated genetic control needle according to the above needle (14), characterized in that a ceramic-coated area of the metal needle is at least a respective portion of an outer and an inner surface thereof that contacts a cell nucleus in tissue of an organism, and that the ceramic film has an arithmetic mean surface roughness Ra of 1.5 µm or less.
25. A ceramic-coated genetic control needle according to the above needle (24), characterized in that the coverage of the insulating ceramic film on the inner surface of the needle extends at least 10 µm from a tip of the needle.
26. A ceramic-coated genetic control needle according to the above needle (24) or (25), characterized in that the ceramic film has a thickness of 0.05 to 5.0 µm.
27. A ceramic-coated genetic control needle according to any one of the above needles (24), (25) or (26), characterized in that the ceramic film comprises at least one selected from the group consisting of nitrides, carbides or oxides of Al, B, Si, Cr and Ti.
28. A ceramic-coated genetic control needle according to any one of the above needles (24) to (27), characterized in that a substrate metal of the needle is stainless steel or high tension steel.
29. A ceramic-coated genetic control needle according to any one of the above needles (24) to (28), characterized in that the needle has a diameter φ of 0.0005 to 0.5 mm.
30. A fabrication method for ceramic-coated medical appliance or biopsy appliance characterized in that an insulating ceramic coating film is formed by dry plating at least on a metal portion of a metallic medical appliance or biopsy appliance that directly contacts an organism.
31. A fabrication method for ceramic-coated medical appliance or biopsy appliance according to the above method (30), characterized in that 5 to 500 sccm of O₂ is introduced in a coating atmosphere in the latter stage of the dryplatingprocess, thereby offering a ceramic-coated medical appliance or biopsy appliance having excellent insulating performance, adhesion and wear resistance and featuring a resistance ρ of 10⁵ Ω·m or more at least at a top surface of the ceramic film.
32. A fabrication method for ceramic-coated medical appliance or biopsy appliance according to the above method (30) or (31) , characterized in that the top surface of the ceramic film has a resistance ρ of 10⁹ Ω·m or more.
33. A fabrication method for ceramic-coated medical appliance or biopsy appliance according to any one of the above methods (30) , (31) or (32), characterized in that the ceramic film comprises at least one selected from the group consisting of nitrides, oxides or carbides of Al, B, Si, Cr and Ti.
34. A fabrication method for ceramic-coated medical appliance according to any one of the above methods (30) to (33), characterized in that the medical appliance is any one of biopsy forceps, forceps, surgical scissors and a surgical knife.
35. A fabrication method for ceramic-coated medical appliance according to any one of the above methods (30) to (33), characterized in that the medical appliance is an injection needle or puncture needle.
36. A fabrication method for ceramic-coated medical needle according to the above method (35) , characterized in that the metal needle is set in parallel with an approaching direction of deposition particles as presenting a tip thereof against the approaching deposition particles and the ceramic film is formed at least on the tip portion of the needle by dry plating.
37. A fabrication method for ceramic-coated medical needle according to the above method (36), characterized in that the ceramic film is an insulating ceramic film having a resistance ρ of 10⁵ Ω·m or more.
38. A fabrication method for ceramic-coated medical needle according to the above method (36) or (37), characterized in that the ceramic film has a thickness of 0.05 to 5.0 µm.
39. A fabrication method for ceramic-coated medical needle according to any one of the above methods (36),(37) or (38), characterized in that the ceramic film comprises at least one selected from the group consisting of nitrides, carbides or oxides of Al, B, Si, Cr and Ti.
40. A fabrication method for ceramic-coated medical needle according to any one of the above methods (36) to (39), characterized in that a substrate metal of the coated needle is stainless steel or high tension steel.
41. A fabrication method for ceramic-coated medical needle according to the above method 35, characterized in that the insulating ceramic film is formed by magnetron sputtering process using, as a sputter target, at least one selected from the group consisting of nitrides, carbides or oxides of Al, B, Si, Cr and Ti while preventing abnormal discharge by disposing ahigh-plasma atmosphere forming magnet and an RF device around the target, thereby overlaying the ceramic film on a part or the whole of an outer surface of the needle and on a portion of an inner surface thereof at least 1 mm inward from a tip of the needle.
42. A fabrication method for ceramic-coated medical needle according to the above method (41), characterized in that two of the sputter targets are disposed for forming the insulating ceramic film based on a W-cathode system.
43. A fabrication method for ceramic-coated medical needle according to any one of the above methods (41) to (42), characterized in that the metal needle is set in parallel with an approaching direction of deposition particles as presenting its tip against the approaching deposition particles.
44. A fabrication method for ceramic-coated medical needle according to any one of the above methods (41),(42) or (43), characterized in that the supply of reaction gas is suspended in an initial stage of the magnetron sputtering process.
45. A fabrication method for ceramic-coated genetic control needle according to any one of the above methods (30) to (33), characterized in that the biopsy appliance is a genetic control needle.
46. A fabrication method for ceramic-coated genetic control needle according to the above method (45) , characterized in that the metal needle is set in parallel with an approaching direction of deposition particles as presenting a tip thereof against the approaching deposition particles, and that the insulating ceramic film having a resistance ρ of 10⁵ Ω·m or more is formed at least on the tip portion of the needle by dry plating with an interior of the needle maintained at higher vacuum than the outside thereof through differential-pressure evacuation.
47. A fabrication system for fabricating a ceramic-coated genetic control needle comprising a magnetron sputtering apparatus, a sample holder and a differential-pressure high-vacuum chamber in a vacuum vessel of a dry plating system, characterized in that a metal needle as a coating object is set on the sample holder as positioned in parallel with an approaching direction of deposition particles and presenting a tip thereof against the approaching deposition particles, and that a trailing end of the needle is connected with the differential-pressure high-vacuum chamber via a tube for maintaining an interior of the metal needle at higher vacuum than outside thereof by differential pressure vacuum effect while an insulating ceramic film is formed on an outer and an inner surface of the metal needle.

### Brief Description of the Drawings

Fig. 1 is a graphical representation of a relation between the resistance ρ of a ceramic coating and the texture damage degree (TDD);
Fig.2A is a diagram showing biopsy forceps according to the invention;
Fig. 2B is a diagram showing conventional biopsy forceps ;
Fig.3A is a diagram showing forceps according to the invention;
Fig.3B is a diagram showing conventional forceps;
Fig.4A is a diagram showing surgical scissors according to the invention;
Fig.4B is a diagram showing conventional surgical scissors;
Fig. 5 is a schematic diagram showing apreferredmagnetron sputtering system used for the fabrication of a ceramic-coated needle according to the invention;
Fig.6A is a diagram showing a setting mode of puncture needles on a sample holder;
Fig.6B is an enlarged sectional view showing a tip of the puncture needle;
Fig. 7 is a transmission electron microscope (TEM) image of a cut-off nucleus;
Fig.8 is a schematic diagram showing another preferred magnetron sputtering apparatus used for the fabrication of the ceramic-coated needle according to the invention;
Fig.9 is a diagram showing details of a procedure of differential-pressure high-vacuum evacuation and a process chamber;
Fig.10 is an enlarged view showing a tip of a metal needle;
Fig.11 is a schematic diagram showing still another preferred magnetron sputtering system used for the fabrication of the ceramic-coated needle according to the invention;

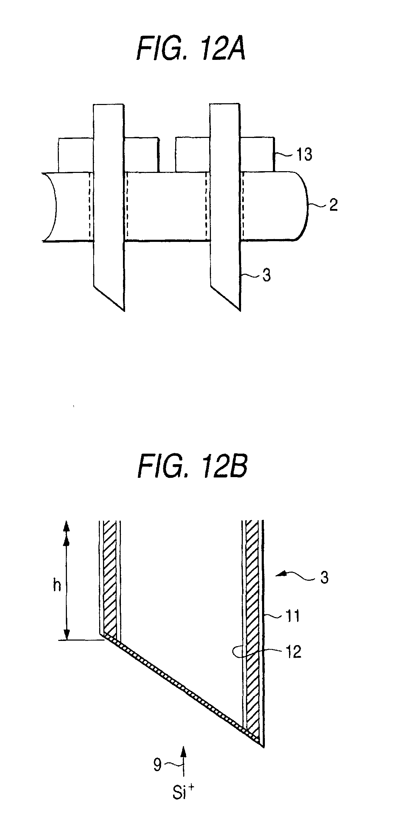

Fig.12A is a diagram showing a setting mode of puncture needles on the sample holder;
Fig.12B is an enlarged sectional view showing a tip of the puncture needle;
Fig.13 is a graphical representation of a relation between the resistance ρ and the amount of introduced oxygen gas;
Fig.14 is a graphical representation of the results of elementary analysis measured from top in the film thickness direction by GDS;
Fig.15A is a graphical representation of a roughness profile (arithmetic mean roughness Ra) of an outer surface of a tip portion of a needle prior to SiNₓ ceramic coating;
Fig.15B is a graphical representation of a roughness profile (arithmetic mean roughness Ra) of the outer surface of the tip portion of the needle subjected to the SiNₓ ceramic coating;
Fig.16A is a graphical representation of a roughness profile (arithmetic mean roughness Ra) of an inner surface of the tip portion of the needle prior to the SiNₓ ceramic coating; and
Fig.16B is a graphical representation of a roughness profile (arithmetic mean roughness Ra) of the inner surface of the tip portion of the needle subjected to the SiNₓ ceramic coating.

### Best Mode for Carrying Out the Invention

The inventors have made intensive studies to solve the aforementioned problem associated with "Background Art" and found that all the ceramic materials are not necessarily usable as a coating ceramic but the usable ceramic material must be an insulating material of high resistance ρ.

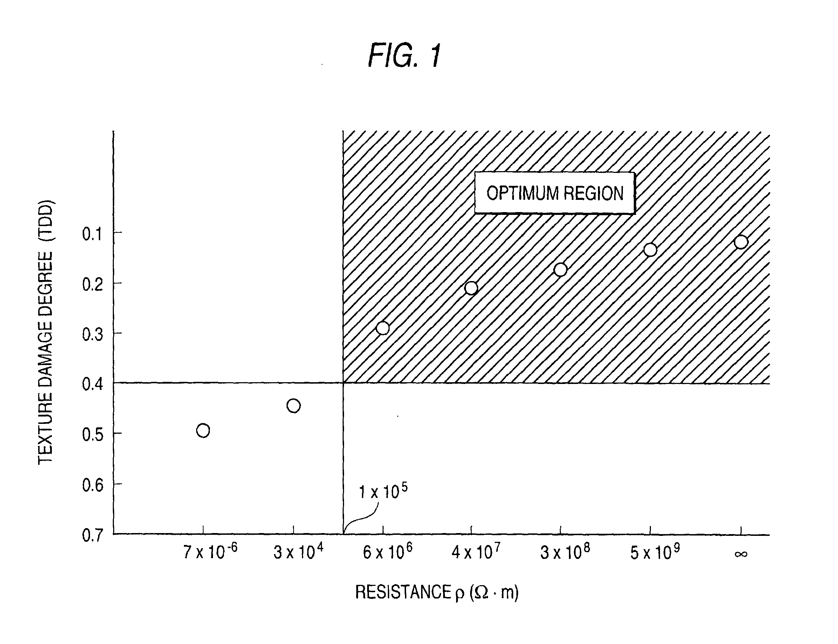

Fig.1 shows the results of an examination of effects on tissues of an organism. The examination used puncture needles formed with ceramic films of different resistances ρ on respective surfaces of stainless steel substrates thereof. The results are represented by a relation between the resistance ρ and the texture damage degree (TDD: histopathological examination by microscopic observation). The texture damage degree (TDD) was determined as follows. Each photographic image of sliced cell tissues was image processed to determine a roughness profile at cut face of the tissues. An arithmetic mean roughness of the roughness profile was found based on JIS B0633 (ISO4288) and then applied to a conversion expression "TDD=0.0563Ra-0.0911" to give a TDD value. The conversion expression was defined based on conditions that cell tissues sliced with a conventional stainless-steel puncture needle had a mean surface roughness Ra of 10.5 µm at cut face a TDD value of 0.5, and that cell tissues sliced with a ceramic-coated needle having a resistance ρ of ∞ had a minimum surface roughness Ra of 3.4 µm at interface and a TDD value of 0.1. It is considered that with the TDD value of 0.40 or less, or preferably of 0.35 or less, there is no unfavorable effects on the tissues of organism due to damages, such as destruction or tear-off.

As shown in Fig.1, good results of TDD values of 0.40 or less were achieved by the use of coating ceramics having resistances ρ of at least 10⁵ Ω·m. In Fig.1, a stainless-steel puncture needle free from the ceramic coating had a resistance ρ of 7×10⁻⁶ whereas a puncture needle coated with a TiN ceramic film had a resistance ρ of 3×10⁴. The resistance is defined herein to mean a volume resistance determined by the four probe method of resistivity measurement according to ASTMD-991.

The invention adopted a ceramic coating process in a high-vacuum, high-plasma atmosphere thereby achieving high adhesion between a high-precision worked metal needle and an insulating ceramic thin film.

Specifically, the invention is characterized by forming a complex structure of iron matrix and a ceramic film combined via a mixed layer of these (including functionally gradient properties) and advantageously capitalizing on individual properties of the components.

The invention will be described in detail as below.

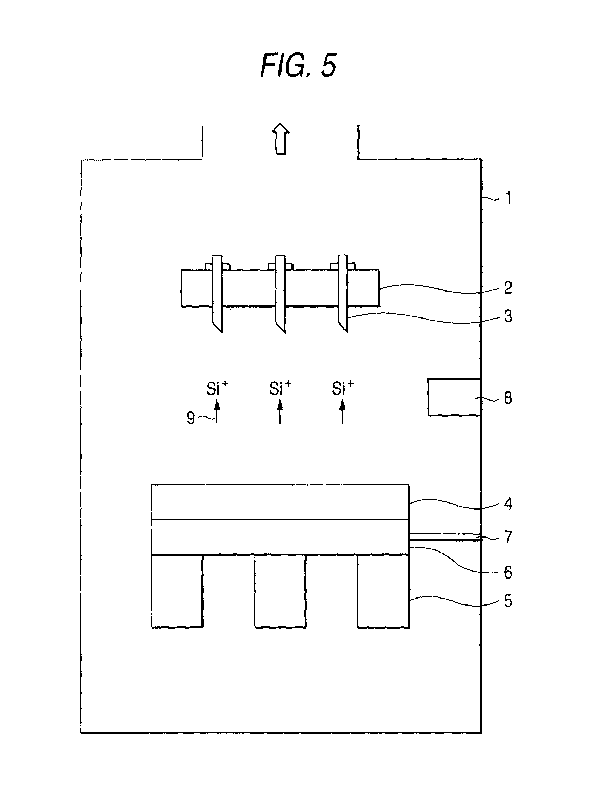

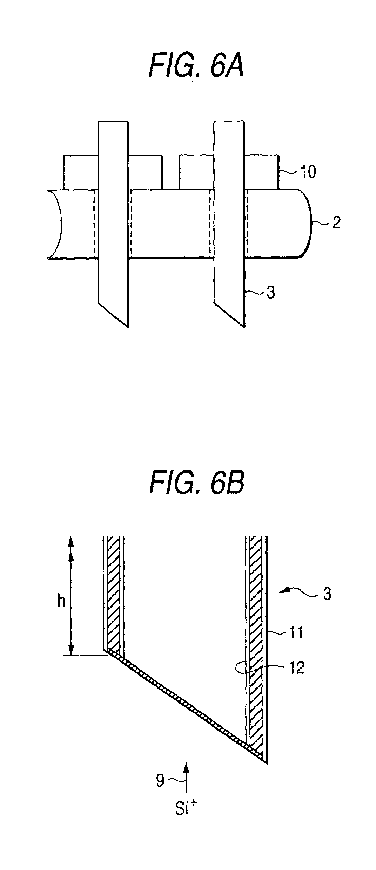

Fig.5 schematically shows a preferred magnetron sputtering system used for the fabrication of a ceramic-coated needle according to the invention. Fig. 6A is a diagram showing a setting mode of metal puncture needles as a material, whereas Fig.6B is an enlarged sectional view of a tip portion of the puncture needle coated with a ceramic film.

Referring to the figures, a reference numeral 1 denotes a vacuum vessel, a numeral 2 denoting a sample holder, a numeral 3 denoting ametal puncture needle. A reference numeral 4 denotes ferrosilicon as a target, a numeral 5 denoting a magnet, a numeral 6 denoting a copper plate interposed between the target and the magnet, a numeral 7 denoting a water cooling pipe for the copper plate. In addition, a numeral 8 denotes an inlet port for a reaction gas, a numeral 9 denoting ionized Si-particles, a numeral 10 denoting a fixing jig for the puncture needles, and numerals 11 and 12 denoting a respective ceramic film formed on an outer surface and an inner surface of the puncture needle 3.

According to Fig. 5, the ionized Si-particles 9 react with nitrogen gas as the reaction gas while moving straight toward the metal puncture needle 3 fixed on the sample holder 2, thereby forming SiNₓ. Thus, SiNₓ is deposited on the surface of the puncture needle 3.

The invention is characterized in that in the above vapor deposition process for forming the deposition particles, i.e., an SiNₓ ceramic, the puncture needle 3 is positioned in parallel with an approaching direction of the deposition particles as presenting a tip thereof against the approaching deposition particles.

Such a setting mode of the puncture needles effectively permits the deposition particles to enter and adhere to interiors of the puncture needles 3. Hence, as shown in Fig.6B, the insulating ceramic film 12 is effectively formed on the inner surface of the puncture needle and, of course, on the outer surface thereof.

It is noted here that the ceramic film on the puncture needle need not cover the whole of the outer surface thereof but only need to cover at least an area which contacts the tissues of an organism during use.

In this respect, there is a similar requirement for the inner surface of the puncture needle, which need be coated with the ceramic film over an area (represented by 'h' in Fig.6B) inwardly extending at least 1 mm from the needle tip (bottom of aperture). This is because the collected tissue slice may sometimes sustain the aforesaidnegative effects unless the inner surface of the puncture needle has an coverage of the insulating ceramic film inwardly extending at least 1 mm from the needle tip. Preferably, the coverage of the ceramic film may inwardly extend about 3 to 10 mm from the needle tip (aperture bottom).

Next, the reason for limitation of the invention will be explained as below.

According to the invention, the ceramic film featuring excellent insulation, adhesion and wear resistance is formed onmedical appliances attheirmetal portions directly contacting any part of the organisms, the medical appliances including medical needles such as injection needles, puncture needles, and suture needles; biopsy forceps; forceps; surgical scissors; surgical knives and the like.

### (1) Coating Substrate

A substrate for medical appliances may be any of metal materials but particularly preferred are stainless steel and high tension steel.

The reason is because stainless steel is free from surface rust and easy to high-precision work. In particular, ferritic stainless steel may be advantageously used.

Preferred chemical compositions of a typical ferritic stainless steel are C: 0.01 wt% or less, Si: 1.0 wt% or less, Mn: 2.0 wt% or less, P: 0.08 wt% or less, S: 0.02 wt% or less, Cr: 10-35 wt%, and N: 0.10 wt% or less, with the remainder being Fe and unavoidable impurities. The ferritic stainless steel may further contain one or two or more types selected from the group consisting of Al: 0.3 wt% or less, Ni: 1.0 wt% or less, Mo: 3.0 wt% or less, Ti: 1.0 wt% or less, Nb: 1.0 wt% or less, V: 0.30 wt% or less, Zr: 1.0 wt% or less, Cu: 1.0 wt% or less, W: 0.30 wt% or less, and B: 0.01 wt% or less.

Preferred chemical compositions of a typical high tension steel are C: 0.01 wt% or less, Si: 1.0 wt% or less, Mn: 2.0 wt% or less, P: 0.08 wt% or less, and S: 0.02 wt% or less, with the remainder being Fe and unavoidable impurities.

In a case where the stainless steel is used as the substrate to fabricate the medical appliances, for example, the following procedure may be taken. A stainless steel material is subj ected to continuous casting process, followed by hot rolling, cold rolling and bright annealing, and then high-precision worked into any of various desired shapes. These steps may be carried out according to the conventional techniques.

In the case of the high tension steel, a commonly taken procedure includes the steps of continuous casting; hot rolling, cold rolling and annealing; welding opposite ends of sheet metal; and working the sheet metal into round pieces.

### (2) Ceramic Film

Metal surfaces of the resultantmedical appliances, which include the medical needles such as injection needles, puncture needles and suture needles; biopsy forceps; forceps; surgical scissors; and surgical knives, are cleaned by ultrasonic cleaning or electrolytic polishing and then, formed with the ceramic film. It is important to use an insulating ceramic having a resistance ρ of at least 10⁵ Ω·m. This is because the ceramics having the resistance ρ of at least 10⁵ Ω·m can fully eliminate the negative effects of damages, such as destruction or tear off, on the sliced tissues or cellular tissues at the lesion site that are contacted by the medical appliances.

Advantageous ceramics having the resistance p of at least 10⁵ Ω·m may be at least one selected from the group consisting of nitrides, carbides or oxides of Al, B, Si, Cr and Ti.

A preferred thickness of the ceramic film is in the range of 0.05 to 5.0 µm, because a ceramic film having a thickness of at least 0.05 µm assures a sufficient insulating performance. On the other hand, a ceramic film having a thickness of 5.0 µm or less is easier to attain good adhesion to the substrate and besides, obviates increased costs associated with the coating process. The film thickness was measured as follows, using a Stylus coating thickness tester Alpha-step 200 (commercially available from Tencor Instrument Inc). The ceramic film was overlaid on a part of a glass slide to define a coated region and a non-coated region, and the film thickness was determined from a difference between levels of the coated region and the non-coated region.

### (3) Ceramic Film Coating Process

The ceramic film coating process is not particularly limited but an advantageous coating process is dry plating. Althoughthemostpreferredprocess ismagnetronsputteringwhich provides high ionization and high-speed film formation, other applicable processes include known PVD coating processes such as RF (Radio Frequency) ionplating (hereinafter, simply referred to as "RF process"), hollow cathode electro-discharge forming, and arc discharge forming; and CVD coating processes; and high-plasma CVD coating processes.

The followings are preferred conditions of ceramic film forming by magnetron sputtering.

In the case of an SiNₓ coating using ferrosilicon as a target, for example, optimum coating conditions are input power: 5-30 kW, vacuum degree: 0.8-3×10⁻³ Torr, Ar gas: 50-1000 sccm, and N₂ gas: 50-1000 sccm (Standard Cubic Centi Meter per Minute).

### (4) Ceramic Coated Portion

The medical appliances of the invention, which include the medical needles such as injection needles, puncture needles and suture needles; biopsy forceps; forceps; surgical scissors; surgical knives and the like, need not have the ceramic film coating on their overall surfaces. The ceramic film only need be formed at least on each portion directly contacting the tis sues of an organism during use.

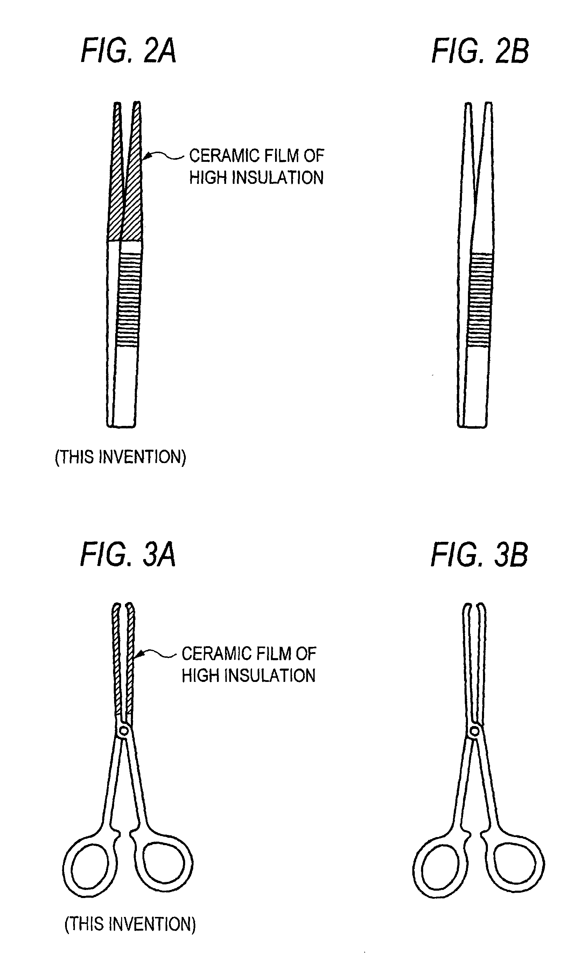

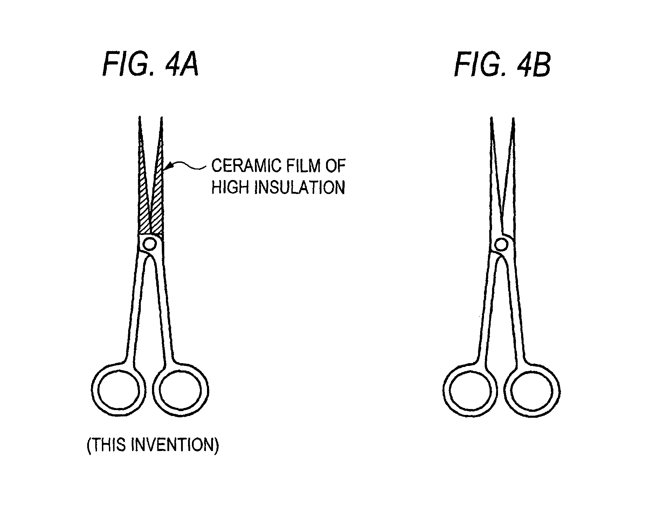

As specific examples of the invention, biopsy forceps, forceps and surgical scissors are shown in Figs.2A, 3A and 4A, respectively. It is noted that Figs.2B, 3B and 4B show the current medical appliances, respectively.

The needles as the subject matter of the invention are not limited to the aforesaid puncture needles and injection needles but may include any medical needles punctured into the organisms, that are used for inj ection of a contrast medium or acupuncture treatment, for example.

Unfortunately, the above coating process has another problem that a ceramic film of excellent insulating performance cannot be attained.

Hence, the inventors have made an intensive study to achieve the improvement and discovered that the insulating performance of the ceramic film can be effectively enhanced by increasing the O₂ concentration of the coating atmosphere of the dry plating process.

Specifically, when the coating process was carried out in an O₂-enriched atmosphere, the resultant ceramic film, which was based on Al, B or Si, had quite an excellent insulating performance with resistance ρ ≥ 10⁹ Ω·m.

Furthermore, even in a ceramic film based on Cr or Ti, which could not achieve a resistance ρ of 10⁵ Ω·m or more according to the conventional techniques, an excellent insulating performance with resistance ρ≥10⁵ Ω·m was attained by increasing the O₂ concentrations of the coating atmosphere.

However, it was also found that in a case where the coating atmosphere was increased in the O₂ concentrations at an initial stage of the dry plating process, the resultant ceramic film was decreased in adhesion.

Therefore, the invention specifies that the O₂ concentrations of the coating atmosphere is increased only in the latter half period of the dry plating process.

The latter half period of the coatingprocess to introduce the oxygen gas in the coating atmosphere is a stage when a percentage of iron contained in the hybrid layer of iron matrix and ceramic is decreased to 20 wt% or less (preferably 10 wt% or less). If this stage is expressed in terms of a thickness of a formed ceramic film, the corresponding film thickness is equal to 10 to 50 % of the total film thickness. In a case where the O₂ concentration of the coating atmosphere was increased in the former stage of the coating process when the percentage of ion contained in the mixed layer of iron matrix and ceramic was in excess of 20 wt%, the resultant ceramic film was decreased in adhesion and hence, the resistance ρ ≥ 10⁵ Ω·m could not be achieved.

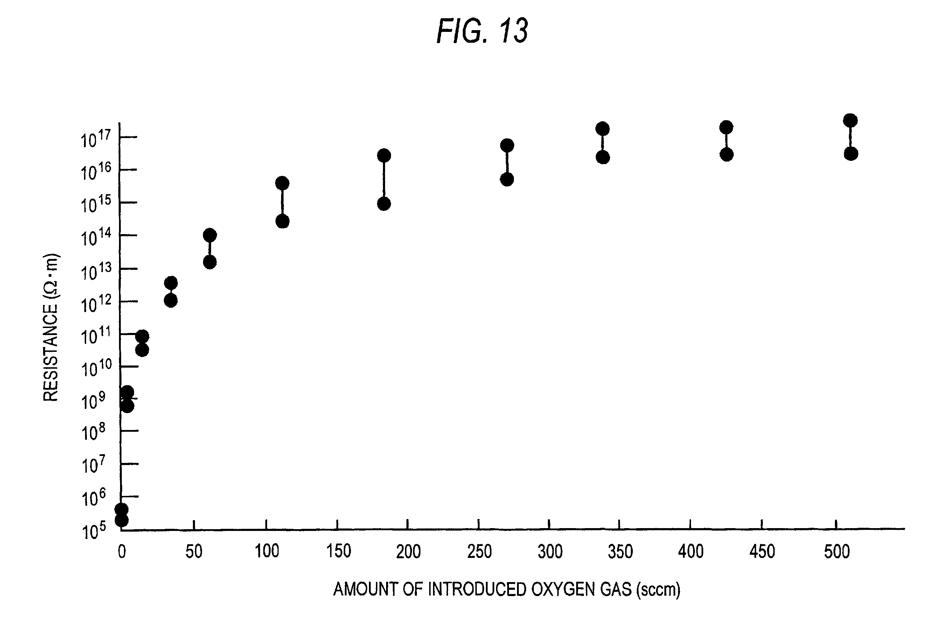

Fig.13 is a graphical representation of a relation between the resistance ρ of a SiNₓ ceramic-coated puncture needle and the amount of introduced oxygen gas, the SiNₓ ceramic film formed in thickness of 1.0 µm on the puncture needle surface by magnetron sputtering. It is noted that the oxygen gas was introduced only in the latter stage of the magnetron sputtering process (surface coating portion: 0.5 µm in thickness).

As seen from Fig.13, the introduction of oxygen gas dramatically increases the resistance ρ. Particularly, when the amount of introduced oxygen gas is 5 sccm or more, the resistance ρ is increased to 10⁹ Ω·m or more, and when the amount of introduced oxygen gas is 50 sccm or more, the resistance p is significantly increased to 10¹³ Ω·m or more.

However, with the amount of introduced oxygen gas in excess of 500 sccm, the increasing effect of the resistance p reaches saturation.

Accordingly, the invention limits the introduction of oxygen gas into the coating atmosphere in the latter stage of the dry plating process to the range of 5 to 500 sccm or preferably of 50 to 500 sccm.

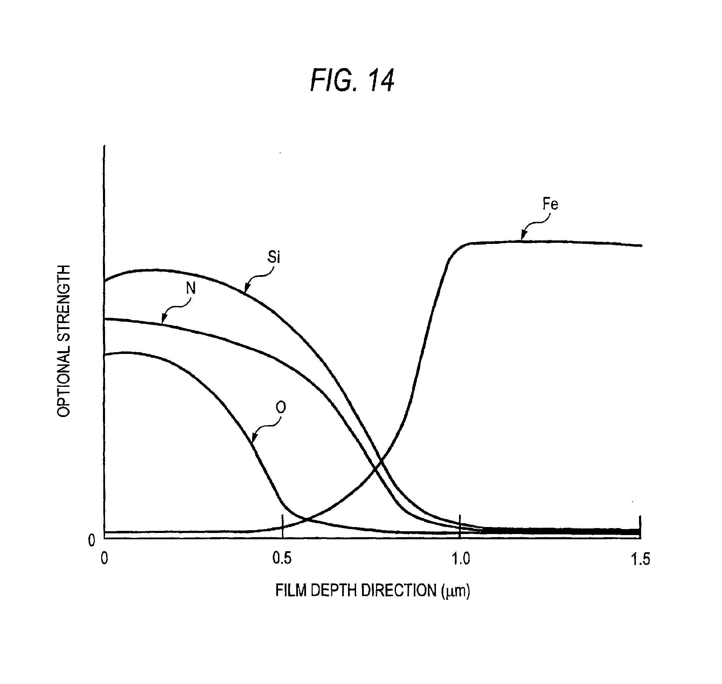

Fig.14 graphically shows the results of analysis of Fe, N, O and Si in the thickness direction of the SiNₓ ceramic film as determined by glow discharge analysis.

As seen in Fig.14, the concentrations of each component varies in the thickness direction. Particularly, O is rich in a 0.5 µm deep region from surface. This indicates that the ceramic film is functionally gradient in the thickness direction so that the ceramic coating film of good adhesion, insulating performance and wear resistance is obtained.

As mentioned above, an advantageous ceramic film to be formed may include at least one selected from the group consisting of nitrides, oxides or carbides of Al, B, Si, Cr and Ti.

The thickness of the ceramic film may preferably be in the range of 0.05 to 5.0 µm, because the ceramic film having a thickness of at least 0.05 µm assures the sufficient insulating performance. On the other hand, the ceramic film having a thickness of 5.0 µm or less is easier to attain good adhesion to the substrate andbesides, obviates increased costs associated with the coating process.

In the dry plating process according to the invention, a single element (metal element) may be used as the target in a case where the ceramic film to be formed is a nitride or carbide. In a case where the ceramic film to be formed is an oxide, it is preferred to use an oxide as the target.

In the formation of the ceramic coating film, an RF (Radio Frequency) device with magnets disposed around the target may advantageously be used for accomplishing high-plasma atmosphere and then good resistance.

The medical appliances of the invention, which include the medical needles such as injection needles, puncture needles and suture needles; biopsy forceps; forceps; surgical scissors; surgical knives and the like, need not have the ceramic film coating on their overall surfaces. The ceramic film only need be formed at least on each portion directly contacting the tissues of an organism during use.

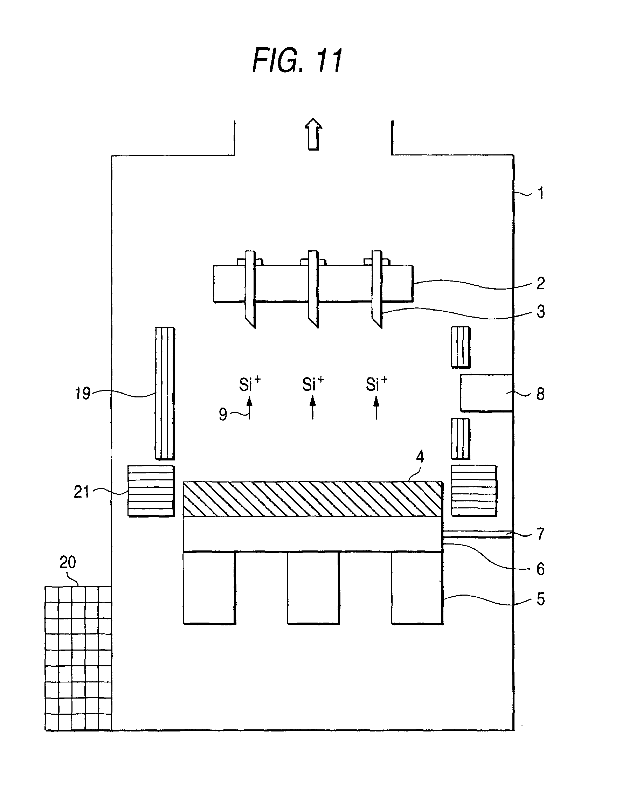

Fig. 11 shows another system according to invention, which provides a favorable plasma atmosphere free from abnormal discharge. According to Fig.11, the deposition particles (ionized Si-particles) 9 react with O₂ gas as the reaction gas while moving straight toward the metal puncture needles 3 fixed on the sample holder 2, so as to be deposited as SiO₂ on the surfaces of the puncture needles 3. It is noted that all the evaporated particles from the target are not ionized Si particles but some of them are SiO₂ particles, not separated into Si and O₂, to be deposited on the metal puncture needles 3 as they are.

According to the invention, the insulating material such as SiO₂ is used as the target, which is provided with a high-plasma atmosphere forming magnet 21 and an RF device 19 in its vicinity, whereby the high-plasma atmosphere free from abnormal discharge can be produced. Therefore, the puncture needle having an excellent insulating performance can be fabricated without decreasing the adhesion of the ceramic film. Incidentally, a reference numeral 20 denotes a power source for the RF device.

The invention employs two sputter targets and adopts W-cathode system for alternately discharging these targets, thereby more effectively preventing the abnormal discharges from the target surfaces. In addition, the high-speed film formation is provided simultaneously.

As mentioned above, the invention uses the insulating material as the target. Hence, the adhesion to the substrate is somewhat decreased as compared with a case where a common metal/semi-metal target is used. However, the invention can obtain the ceramic film far more excellent in the insulating performance than a process wherein a reactive ceramic film is formed in the course of the process using the metal/semi-metal target.

A preferred thickness of the ceramic film is in the range of 0.05 to 5.0 µm, because a ceramic film having a thickness of at least 0. 0 5 µm assures the sufficient insulating performance. On the other hand, a ceramic film having a thickness of 5.0 µm or less is easier to achieve good adhesion to the substrate and besides, obviates increased costs associated with the coating process.

The coating process of the ceramic film according to the invention is not particularly limited, as described above. However, an advantageous coating process is dry plating. As to the dry plating, it is preferred to adopt magnetron sputtering which provides the atmosphere of high ion concentration and high-speed film forming, and which facilitates the control of ceramic film thickness and the film coating process.

In this case, it is preferred to provide the high-plasma atmosphere forming magnet 21 and the RF device 19 around or above the cathodes for stable execution of the coating process in the high-plasma atmosphere free from abnormal discharge. In this case, the W-cathode system alternately discharging two sputter targets may be adopted for more effective prevention of the abnormal discharge.

The followings are preferred conditions for forming the ceramic film by magnetron sputtering.

For instance, a sintered SiO₂ target may be used for SiO₂ coating whereas a sintered SiNₓ targetmay be used for SiNₓ coating. In such cases, optimum coating conditions are input power: 5-50 kW, vacuum degree: 0.8×10⁻⁴-3×10⁻³ Torr, Ar gas rate: 50-500 sccm, and reaction gas rate (O₂ or N₂ gas): 50-2000 sccm.

In a case where the insulating target is used with the flowing reaction gas and all the particles from the target are deposited as a film of nitride, carbide or oxide, the resultant film has a somewhat lower adhesion to the needle as the substrate than a film formed from ionized particles from a metal target. Hence, a more advantageous approach may be taken to prevent the deterioration of adhesion, the approach wherein the supply of reaction gas such as O₂ or N₂ is suspended in the initial stage of the coating process so that the coating process takes place in the presence of a certain amount of ionized particles.

The needles as the subject matter of the invention are not limited to the aforesaid puncture needles, inj ection needles and suture needles and may include any medical needles punctured into the organisms, that are used for injecting the contrast medium, for example.

As mentioned above, the invention is characterized in that the ceramic insulating material, such as sintered SiO₂, rather than the metal material is used as the target in order to form the ceramic coating film having the excellent insulating performance, and that the high-plasma atmosphere forming magnet 21 and the RF device 19 are disposed in vicinity of the cathodes for overlaying the ceramic film of high insulation in the favorable plasma atmosphere free from abnormal discharge.

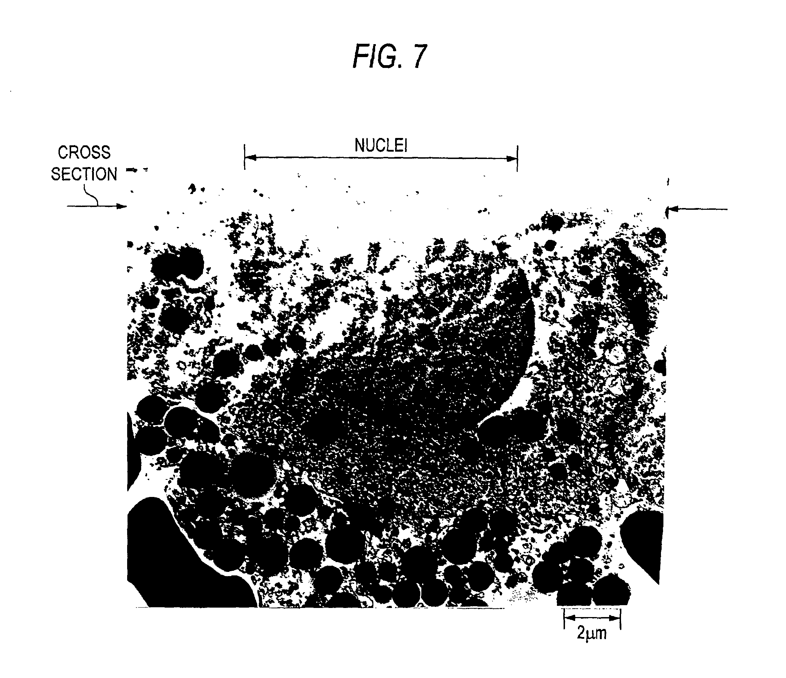

The inventors used the above ceramic-coated puncture needle of the invention to collect rat hepatic tissues, which were observed in details with a transmission electronmicroscope. As a result, the following new facts were discovered.
a) The surface of the tissues severed with the ceramic-coated puncture needle is much smoother than that of tissues severed with the ordinary stainless-steel puncture needle.
b) A case was observed wherein a part of a nucleus of a cell collected with the ceramic-coated puncture needle was cut off as shown in a transmission electron microscopic image of Fig. 7.

Thus, the ceramic-coated needle with the ceramic film of high insulating performance have extremely decreased negative effects, such as destruction or tear-off, on the tissues or cells of the organism.

Hence, the inventors investigated the applicability of the invention to a wide range of technical fields in order to find applications of the above features of the ceramic-coated needle in other fields than the medical field.

Consequently, the inventors found that the invention provides excellent effects in the field of biopsy or particularly genetic control.

In the field of genetic control, glass or ceramic needles have conventionally been used for cutting off a cellular nucleus or injecting an immunological solution into the nucleus. Unfortunately, these needles have the following drawbacks.
1) A genetic control needle for cutting off the cellular nucleus or injecting the immunological solution or the like into the nucleus must be very fine. For instance, the nuclei have diameters on the order of 2 to 5 µm and hence, the needle must be commensurate in size in order to perform the genetic control. However, it is extremely difficult to form such a fine needle from glass or ceramic and hence, the minimum possible needle size that is achieved by the prior art is 0.7 µm or so.
2) Conventionally, the needle having the diameter on the order of 0.7 µm has been used for cutting off the nucleus or injecting the immunological solution. Unfortunately, such a needle detrimentally has a lower cutting performance so as to encounter resistance from a nuclear envelope as a protective film of the nucleus, failing to accomplish precise cut-off of the nucleus or accurate injection of the immunological solution.
3) Since glass or ceramic is so fragile that it is naturally difficult to form a fine needle from glass or ceramic. In addition, such a glass or ceramic needle tends to break easily when used, and hence are difficult to handle.

In this connection, the inventors fabricated a novel ultra-fine ceramic-coated needle for genetic control based on the above findings and tried the resultant needle in the cut off of nuclei or injection of the immunological solution or the like.

As a result, it was demonstrated that such a ceramic-coated needle was capable of precisely cut off a part of a nucleus in a cell, permitting the genetic control without causing damaging effects, such as cell destruction or tear-off, on the cells or tissues around the penetrated needle.

The invention is based on the above finding.

The invention will hereinbelow be described in detail.

An ultra-fine needle of an equivalent size to that of the nucleus is required for achieving the "genetic control via cut-off of the cell nucleus or injection of the immunological solution" as the object of the invention.

The cellular nuclei include egg cells (diameter: about 25 µm) as a larger one and normal cell nuclei (diameter: about 2 to 5 µm) as a smaller one.

The reason for the limitation of the inventive ceramic-coated genetic control needle will be explained as below.

### (1) Diameter of Ceramic-Coated Genetic Control Needle

In order to inject the immunological solution into a cell nucleus, the diameter of the needle is not necessarily smaller than that of the nucleus but may be of a size such that a tip of the needle can cut into the nucleus to inj ect the immunological solution. In the light of a precise control of the amount of injected immunological solution, however, it is of course advantageous that the needle has a size about 1/4 to 1/5 of that of the nucleus such as to permit its tip to bodily enter the nucleus.

Therefore, the invention defines a preferred size of the ceramic-coated genetic control needle to be in the range of 0.0005 to 0.5 mm.

### (2) Substrate for Ceramic-Coated Genetic Control Needle

Although any metal materials are usable as a substrate for the ultra-fine needle, particularly preferred is stainless steel.

The reason is because stainless steel is free from surface rust and easy to high-precision work. Above all, ferritic stainless steel may be advantageously used.

In a case where stainless steel is used as the substrate to fabricate the genetic control needle, for example, the following procedure may be taken. A stainless steel material is subjected to continuous casting process, followed by hot rolling, cold rolling and bright annealing, and then high-precision worked into a desired shape. These steps may be carried out according to the conventional techniques.

### Subsequently, the resultant needle is precision worked to a diameter of about 0.0005 to 0.5 mmφ using the advanced high-precision working technique.

### (3) Ceramic Film on Ceramic-Coated Genetic Control Needle

A surface of the resultant needle is cleaned by ultrasonic cleaning or electrolytic polishing and then, coated with a ceramic film. It is important to use an insulating ceramic having a resistance ρ of at least 10⁵ Ω·m. This is because the ceramic having the resistance ρ of at least 10⁵ Ω·m can fully eliminate the negative effects on sliced tissues, cells on a lesion area or a target cell nucleus contacted by the needle.

### (4) Fabrication Method for Ceramic-Coated Genetic Control Needle

Description will hereinbelow be made on a method for overlaying the ceramic film on an outer and an inner surface of the genetic control needle using the dry plating technique.

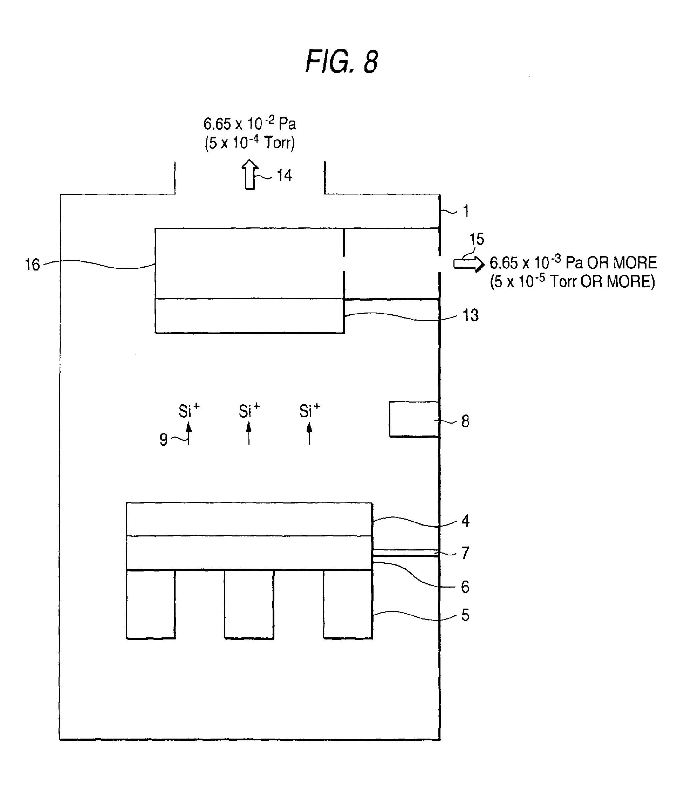

Fig.8 schematically shows a magnetron sputtering system preferably used for the fabrication of a ceramic-coated genetic control needle according to the invention.

In Fig. 8, a reference numeral 1 denotes a vacuum vessel, a numeral 13 denoting a process chamber containing a sample holder andmetal needles, a numeral 4 denoting a ferrosilicon as a target, a numeral 5 denoting a magnet, a numeral 6 denoting a copper plate interposed between the target and the magnet, a numeral 7 denoting a water cooling pipe for the copper plate, a numeral 8 denoting an inlet port for a reaction gas. In addition, an arrow 14 denotes a direction in which the air in the vacuum vessel 1 is evacuated (about 5×10⁻⁴ Torr), an arrow 15 denoting differential-pressure high-vacuum evacuation for maintaining the interior of the metal needles in higher vacuum (at least 5×10⁻⁵ Torr, or at least 6.65×10⁻³ Pa), a numeral 9 denoting ionized Si-particles. A numeral 16 denotes a differential-pressure high-vacuum chamber.

Referring to Fig. 8, the ionized Si-particles 9 react with nitrogen gas as the reaction gas while moving straight toward the metal puncture needles 3 fixed on the sample holder 2 in the process chamber 13, so as to be deposited as SiNₓ on the surfaces of the puncture needles 3.

However, the genetic control needle 3 is so fine as to make the invasion of plasma vapor flow into the needle extremely difficult.

According to the invention, a setting mode of the metal needles 3 is improved while the advanced differential-pressure evacuation technique is utilized to produce a state where a vacuum degree inside of the needle is higher than that outside of the needle. In this sate, the plasma coating process is carried out, allowing the plasma vapor flow to enter the interior of the needles for SiNₓ deposition.

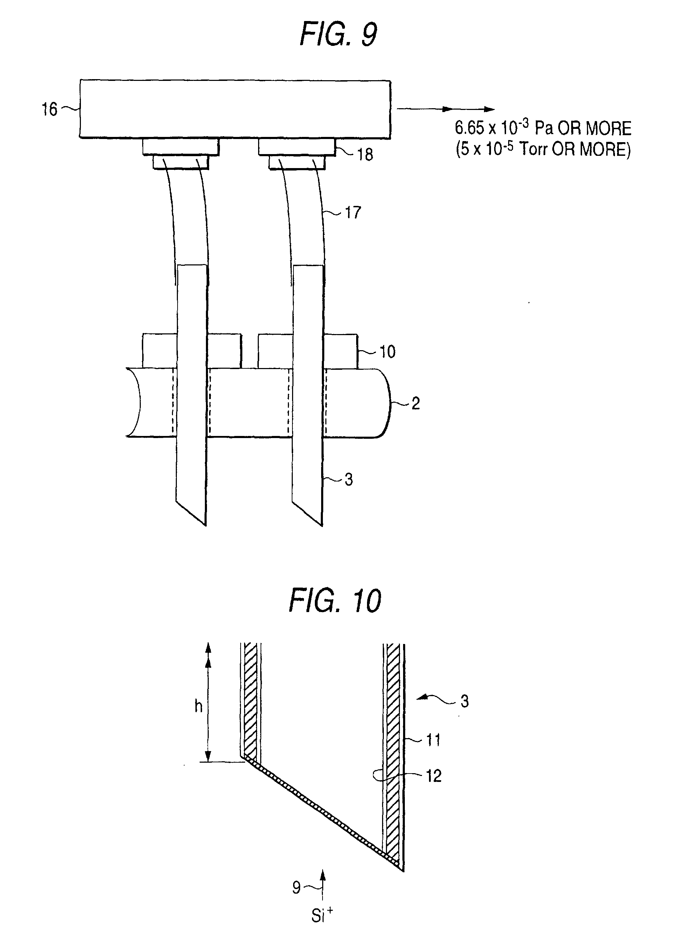

Now referring to Fig.9, a procedure for the differential-pressure high-vacuum evacuation and of the process chamber are described in detail.

In Fig.9, a numeral 3 denotes a metal genetic control needle, a numeral 2 denoting the sample holder, a numeral 10 denoting a fixing jig for the genetic control needles 3, a numeral 17 denoting a tube interconnecting the genetic control needle 3 and the differential-pres surehigh-vacuum chamber 16, a numeral 18 denoting a joint for coupling the tube 17 with the differential-pressure high-vacuum chamber 16.

As shown in Fig.9, a first feature of the invention is that in the vapor deposition process for forming the SiNₓ ceramic film, the metal needle 3 is positioned in parallel with an approaching direction of the deposition particles as presenting a tip thereof against the approaching deposition particles.

Such a needle setting mode effectively permits the deposition particles to enter and adhere to the interior of the metal needle 3.

A second feature of the invention is that the metal genetic control needle 3 is connected with the differential-pressure high-vacuum chamber 16 so that the plasma coating is carried out in a state where the vacuum degree on the inside of the metal needle 3 is made higher than that outside of the needle 3 by the differential-pressure evacuation technique.

The use of such a differential-pressure evacuation technique more effectively permits the deposition particles to enter the interior of the metal needle 3 and to be deposited thereto. As a result, a coverage of the ceramic film coating extends further inwardly of the metal needle 3 than a case where the differential-pressure evacuation technique is not used.

In fact, when the ceramic film was overlaid on the metal needle 3 according to the invention, the insulating ceramic film was effectively formed not only on the outer surface of the metal needle 3 but also on the inner surface thereof, as shown in Fig.10. In the figure, a numeral 11 denotes the ceramic film overlaid on the outer surface of the metal needle 3, whereas a numeral 12 denotes the ceramic film overlaid on the inner surface thereof.

It is preferred here that a difference between the differential-pressure vacuum degrees at the outside and at the inside of the metal needle is at least 4.5×10⁻⁴ Torr, although such a difference may vary depending upon the diameter of the metal needle.

As to an evacuation machine for evacuating the air from the vacuum vessel, a diffusion pump may sufficiently serve the purpose. However, a vacuum pump for achieving the differential-pressure high vacuum must be a pump capable of easily achieving the high vacuum. An example of such a pump includes Crio Pump and Turbo Pump.

The ceramic films on the outer and inner surfaces of the metal genetic control needle need not cover the overall surfaces of the needle. The ceramic film on the outer surface only need to cover at least a portion contacting the tissues of the organism, whereas the ceramic film on the inner surface only need to cover a portion contacting the cell nuclei in the tissues of the organism.

Specifically, the ceramic-coated area of the outer surface is at least 1 mm or preferably 10 mm from the tip of the metal needle, whereas that of the inner surface is at least 10 µm or preferably at least 1 mm from the tip (aperture bottom) of the metal needle (represented by 'h' in Fig.10).

The followings are preferred conditions for forming the ceramic film by magnetron sputtering.

In the case of an SiNₓ coating using ferrosilicon as a target, for example, optimum coating conditions are input power: 5-30 kW, vacuum degree (in vacuum vessel) : 0.8-3×10⁻³ Torr, Ar gas: 50-1000 sccm, and N₂ gas: 50-1000 sccm.

A ceramic film having quite a high insulating performance with resistance ρ of at least 10⁹ Ω·m can be obtained by introducing about 5 to 500 sccm of O₂ gas during the plasma coating process or particularly in the latter stage thereof.

### (5) Surface Roughness Ra of Ceramic-Coated Genetic Control Needle

Smoothing the surface of the ceramic-coated needle significantly reduces the occurrence of morphological defects of cellular nuclei, such as cut face or contact surface tear-off, caused by the needle cutting off the nuclei or injecting the immunological solution.

Therefore, it is preferred to limit the surface roughness Ra of the ceramic film to 1.5 µm or less, or more preferably to 1.3 µm or less. The reason for the limitation will be explained as below.

The aforementioned magnetron sputtering method was used to form a 0.7 µm-thick SiNₓ ceramic film on an outer and an inner surface of a stainless-steel needle having a diameter of 0.1 mm. The SiNₓ ceramic films on the outer and inner surfaces of a tip portion of the needle were examined for the surface roughness.

In this examination, pre-coating surface roughnesses of the outer and inner surfaces of the stainless steel needle were also determined the same way and then, the pre-coating and post-coating surface roughnesses were compared.

The above examination used a surface texture measuring instrument commercially available from Belty Inc. (Germany) and the arithmetic mean surface roughness Rawasdeterminedaccording to JIS B0633 (ISO4288). Since the diameter of the needle was too small to take accurate measurement on the inner surface, a similar SiNₓ ceramic film was formed on a stainless steel needle having a diameter of 1. 5 mm andmeasured. Measurement conditions were cut-off value λc: 5 µm, and measuring range lₙ: 5 mm.

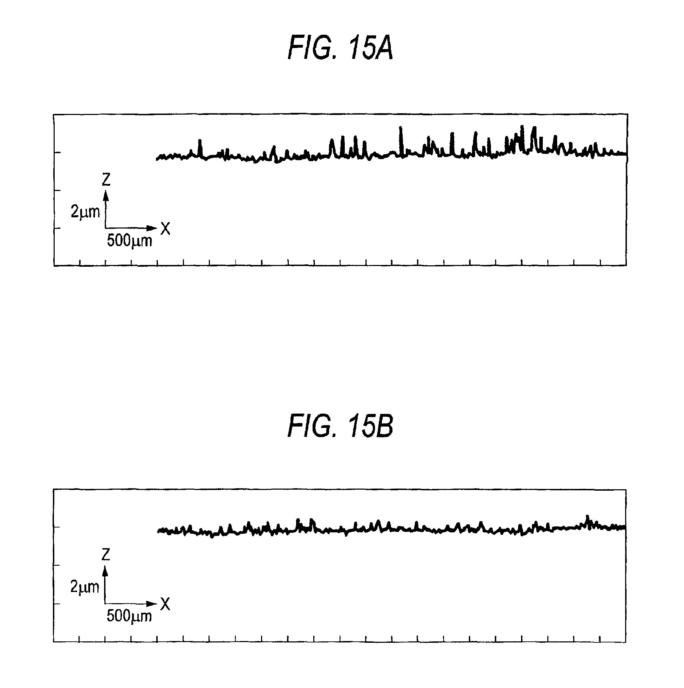

For comparison, Figs.15A and 15B show roughness profiles at the outer surface (arithmetic mean roughness Ra) of the tip portion of a needle before and after the deposition of the SiNₓ film. For comparison, Figs .16A and 16B show roughness profiles at the inner surface (arithmetic mean roughness Ra) of the tip portion of the needle before and after the deposition of the SiNₓ film.

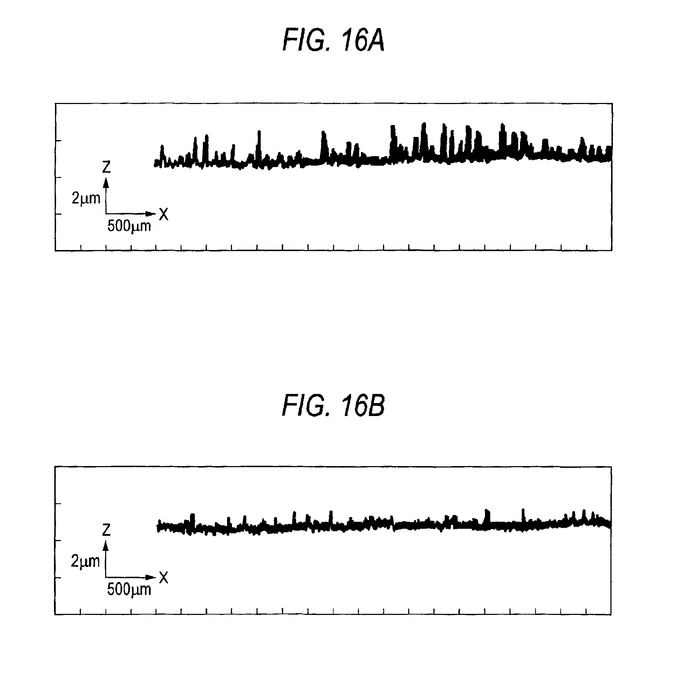

As seen from Figs.15A and 16A, the stainless steel needle has arithmetic mean roughnesses Ra on the order of 2 µm at the outer surface and the inner surface. In contrast, the stainless steel needle formed with the SiNₓ ceramic film is improved in the arithmetic mean roughness Ra to 1.2 µm or less at both the outer and inner surfaces, as seen in Figs.15B and 16B.

Aparticularly effective approach to decrease the surface roughness of the ceramic coating film is to adopt the aforementioned coating method wherein O₂ is introduced in the atmosphere in the latter stage of the dryplatingprocess, thereby imparting the ceramic film with the functionally gradient property in the thickness direction. The coating method provides stable reduction of the surface roughness Ra of the ceramic film to 1.3 µm or less.

As described above, both the outer and inner surfaces of the needle are dramatically smoothed by subj ecting the needle to the SiNₓ ceramic coating process in the atmosphere of high-density plasma. Thus, the use of such a needle results in the smooth cut-off of the cellular tissue or nuclei.

In addition, the smoothed surface of the ceramic coating film contributes to a notable reduction of the occurrence of morphological defects of cellular nuclei, such as cut face or contact surface tear-off, caused by the needle cutting off the nuclei or injecting the immunological solution.

Therefore, the ceramic film of the invention may preferably have a surface roughness Ra of 1.5 µm or less or more preferably of 1.3 µm or less.

### Example 1

A ferritic stainless steel material, containing C: 0.039 mass%, Si: 0.25 mass%, Mn: 0.12 mass%, P: 0.008 mass%, S: 0.012 mass%, and Cr: 18.9 mass% with the remainder being Fe and unavoidable impurities, was subjected to continuous casting process, followed by hot rolling, cold rolling and bright annealing, and then high-precision worked into biopsy forceps, forceps, surgical scissors and a surgical knife.

Subsequently, themedical appliances were supersonically cleaned and subjected to the magnetron sputtering process illustrated in Fig.5 for formation of various ceramic films on the individual portions shown in Figs.2A to 4A. Specifically, the SiNₓ film was formed on the biopsy forceps, the SiO₂ film was formed on the forceps, a BN film was formed on the surgical scissors, and an Al₂O₃ film was formed on the surgical knife. For comparison purpose, a TiN film was formed on the biopsy forceps. All the coating films had a thickness of about 1 µm. Thickness measurement was performed in the aforementioned manner using the Stylus coating thickness tester Alpha-step 200 (commercially available from Tencor Instrument Inc). That is, the film thickness was determined from a difference between levels of the coated region and the non-coated region on the glass slide.

The resultant medical appliances including the biopsy forceps, forceps, surgical scissors and surgical knife were examined for the insulating performance (resistance ρ) and adhesion of the ceramic films thereof. The results are listed in Table 1.

The adhesion of the ceramic films was determined as follows. A ceramic-coated stainless steel sheet was prepared by forming each ceramic film on the stainless steel sheet the same way as the above. Each ceramic-coated steel sheet was wrapped around each of bars of different diameters to evaluate the adhesion based on a minimum diameter of a bar that did not encounter the delamination of the ceramic film. A minimum diameter of 20 mm or less was graded as good adhesion (○), a diameter of more than 20 mm to 30 mm or less was graded as acceptable (Δ) and a minimum diameter of more than 30 mm was graded as poor (×). As described above, the resistance ρ was determined by the four probe method of resistivity measurement. The resistance p of ∞ means a resistance ρ of at least 10⁹ Ω·m.

As shown in Table 1, all the medical appliances according to the invention had quite excellent insulating performance and good film adhesion.

### Example 2

A ferritic stainless steel material, containing in mass percentage C: 0.033 %, Si: 0.20 %, Mn: 0.15 %, P: 0.008 %, S: 0.008%, and Cr: 17.7% with the remainder being Fe and unavoidable impurities, was subjected to continuous casting process, followed by hot rolling, cold rolling and bright annealing, and then high-precision worked into a puncture needle (outside diameter: 2.0 mm, length: 170 mm) , biopsy forceps, forceps, surgical scissors and a surgical knife.

Subsequently, the medical appliances were supersonically cleaned and subjected to the magnetron sputtering process illustrated in Fig. 5 (with the RF device also used in some step) for formation of various ceramic films listed in Table 2 in the high-plasma atmosphere. In the coating process, the former stage was carried out in the normal coating atmosphere, whereas the latter stage (surface coating portion: 0.5 µm in thickness) was carried out with oxygen gas introduced in the atmosphere in different amounts. All the coating films had a thickness of about 1.0 µm. Thickness measurement was taken the same way as in Example 1. The coating conditions were input power: 6 kW, Ar gas: 100 sccm, and N₂ gas: 130 sccm (No.1, 3, 5).

The resultant medical appliances were examined for the insulating performance (resistance ρ) and adhesion of the ceramic films thereof. The results are listed in Table 2.

The adhesion of the ceramic films listed in Table 2 was determined as follows. A ceramic-coated stainless steel sheet was prepared by forming each ceramic film on the stainless steel sheet the same way. Each ceramic-coated steel sheet was wrapped around each of bars of different diameters to evaluate the adhesion based on the minimum diameter of a bar that did not encounter the delamination of the ceramic film. The adhesion of the ceramic films was evaluated based on the same criteria as in Example 1. Similarly to Example 1, the resistance p was determined by the four probe method of resistivity measurement. The resistance ρ = ∞ means a resistance ρ of at least 10⁹ Ω·m.

As shown in Table 2, all the medical appliances according to the invention had quite excellent insulating performance and good film adhesion.

### Example 3

A ferritic stainless steel material (a) containing C: 0.03 mass%, Si: 0.2 mass%, Mn: 0.15 mass%, P: 0.010 mass%, S: 0.010 mass%, and Cr: 18.8 mass% with the remainder being Fe and unavoidable impurities; and an austenite stainless steel material (b) containing C: 0.05 mass%, Si: 0.3 mass%, Mn: 0.20 mass%, P:0.012 mass%, S: 0.011 mass%, Cr: 19.1 mass%, Ni: 9.1 mass% and Mo: 0.25 mass% with the remainder being Fe and unavoidable impurities were each subj ected to continuous casting process, followed by hot rolling, cold rolling and bright annealing, and then high-precision worked into a contrast medium needle having an outside diameter of 0. 8 mm and a length of 200 mm. After supersonically cleaned, the contrast medium needles were set in the magnetron sputtering system shown in Fig. 5 for SiNₓ film formation in the high plasma atmosphere.

The magnetron sputtering process formed the SiNₓ ceramic film in a thickness of 0.8 µm under the conditions of Ar: 100 sccm, and N₂: 65 sccm. Thickness measurement was taken the same way as in Example 1. A coverage area of the SiNₓ film on the outer surface was 70 mm from the needle tip, whereas a coverage area of that on the inner surface was 6 mm from the needle tip (aperture bottom).

Table 3 shows the results of examination on the texture damage degree (TDD) of tissues collected using each of the resultant ceramic-coated contrast medium needles and the adhesion of each of the SiNₓ ceramic films.

The adhesion of the ceramic films was determined as follows. A ceramic-coated stainless steel sheet was prepared by forming each ceramic film on the stainless steel sheet the same way. Each ceramic-coated steel sheet was wrapped around each of bars of different diameters to evaluate the adhesion based on the minimum diameter of a bar that did not encounter the delamination of the ceramic film. The adhesion of the ceramic films was evaluated based on the same criteria as in Example 1. Similarly' to Example 1, the resistance ρ was determined by the four probe method of resistivity measurement. The resistance ρ = ∞ means a resistance ρ of at least 10⁹ Ω·m.

For comparison purpose, Table 3 also shows the results of a contrast medium needle formed from the ferritic stainless steel (a) and free from the ceramic film.

As shown in Table 3, both the ceramic needles according to the invention had good film adhesion and presented good TDD values of 0.15 or less for the collected tissues.

### Example 4

A ferritic stainless steel material containing C: 0.03 mass%, Si: 0.3 mass%, Mn: 0.12 mass%, P: 0.011 mass%, S: 0.009 mass%, and Cr: 16.9 mass% with the remainder being Fe and unavoidable impurities, was subjected to continuous casting process, followed by hot rolling, cold rolling and bright annealing, and then high-precision worked into a needle for biopsy examination (hereinafter, referred to as "biopsy needle") having an outside diameter of 2.1 mm and a length of 170 mm. After supersonically cleaned, the biopsy needle was subjected to the magnetron sputtering process illustrated in Fig. 5 (with the RF device also used in some step) for formation of various ceramic films listed in Table 4 in the high-plasma atmosphere. Thickness measurement was taken the same way as in Example 1. In this process, the biopsy needle was set in the same way as that shown in Fig.5.

Table 4 shows the results of examination on the texture damage degree (TDD) of tissues collected using each of the resultant ceramic-coated needles and the adhesion of the ceramic films. The adhesion of the ceramic films was evaluated based on the same criteria as in Example 1. Similarly to Example 1, the resistance ρ was determined by the four probe method of resistivity measurement. The resistance ρ = ∞ means a resistance ρ of at least 10⁹ Ω·m.

As shown in Table 4, in the case where the insulating ceramic film having a resistance ρ of at least 10⁵ Ω·m was formed according to the invention, the TDD for the collected tissues was reduced to 0.35 or less. Therefore, the use of such needles fully eliminated the negative effects not only on the cells around the needle penetrated into the lesion but also on the sliced tissues.

### Example 5

A ferritic stainless steel material containing C: 0.033 mass%, Si: 0.3 mass%, Mn: 0.13 mass%, P: 0.009 mass%, S: 0.011 mass%, and Cr: 17.9 mass% with the remainder being Fe and unavoidable impurities, was subjected to continuous casting process, followed by hot rolling, cold rolling and bright annealing, and then high-precision worked into a puncture needle having an outside diameter of 0. 9 mm, an inside diameter of 0. 75 mm and a length of 230 mm.

After supersonically cleaned, the needle was set in the magnetron sputtering system shown in Fig.11. for formation of various 1. 0 pm-thick ceramic films in the high-plasma atmosphere. Thickness measurement was taken the same way as in Example 1.

In this process, the puncture needles were set the same way as shown in Fig.11 while each of various materials listed in Table 5 was used as the sputter target. In particular, the W-cathode system was used when the target No.2 or No.4 was used in Table 5.

A coverage area of the ceramic film formed on the outer surface by the magnetron sputtering process was on the order of 50 to 80 mm from the needle tip, whereas a coverage area of that on the inner surface was on the order of 5 to 8 mm from the needle tip (aperture bottom).

Table 5 shows the results of examination on the adhesion and the insulating performance (resistance ρ) of the ceramic films on the ceramic-coated puncture needles.

The adhesion of the ceramic films was determined as follows. A ceramic-coated stainless steel sheet was prepared by forming each ceramic film on the stainless steel sheet the same way. Each ceramic-coated steel sheet was wrapped around each of bars of different diameters to evaluate the adhesion based on the minimum diameter of a bar that did not encounter the delamination of the ceramic film. The adhesion of each ceramic film was evaluated based on the same criteria as in Example 1. Similarly to Example 1, the resistance ρ was determined by the four probe method of resistivity measurement. The resistance ρ = ∞ means a resistance ρ of at least 10⁹ Ω·m.

As shown in Table 5, the ceramic-coated needles according to the invention had quite excellent insulating performance and favorable film adhesion.

### Example 6 (Egg Cell with Large Nucleus (about 25 µm))

A ferritic stainless steel material containing C: 0.030 mass%, Si: 0.22 mass%, Mn: 0.18 mass%, P: 0.009 mass%, S: 0.008 mass%, and Cr: 17.9 mass% with the remainder being Fe and unavoidable impurities, was subjected to continuous casting process, followed by hot rolling, cold rolling and bright annealing, and then high-precision worked into a metal. needle having an outside diameter of 0.2 mm, an inside diameter of 0.15 mm and a length of 100 mm.

After supersonically cleaned, the metal needle was coated with an SiNₓ film by means of the magnetron sputtering system shown in Figs.8 and 9.

The magnetron sputtering process formed a 0.7 µm-thick SiNₓ film under conditions of Ar: 100 sccm, and N₂: 150 sccm. Thickness measurement was taken the same way as in Example 1. A coverage area of the SiNₓ ceramic film on the outer surface was 50 mm from the needle tip, whereas a coverage area of that on the inner surface was 8 mm from the needle tip (aperture bottom).

When the SiNₓ ceramic-coated needle was used to inject an immunological solution into an egg, the tip of the ceramic needle smoothly entered the egg and a predetermined amount of immunological solution could be injected accurately.

The needle had substantially no negative effects on the tissues of the organism, presenting a texture damage degree (TDD) of 0.33.

### Example 7 (Lymphatic Cell with Small Nucleus (about 3 µm))

A ferritic stainless steel material containing C: 0.045 mass%, Si: 0.34 mass%, Mn: 0.25 mass%, P: 0.010 mass%, S: 0.007 mass%, and Cr: 16.9 mass% with the remainder being Fe and unavoidable impurities, was subjected to continuous casting process, followed by hot rolling, cold rolling and bright annealing, and then high-precision worked into a metal needle having an outside diameter of 0.005 mm, an inside diameter of 0.002 mm and a length of 50 mm.

After supersonically cleaned, the metal needle was coated with a 0.5 µm-thick BN film by means of the magnetron sputtering system shown in Figs. 8 and 9 (with the RF device also used in some step). Thickness measurement was taken the same way as in Example 1.

When the BN ceramic-coated needle was used to cut off a cell nucleus, a precise cut-off of the nucleus at an intended site could be achieved.

As a matter of course, the needle had substantially no negative effects on the tissues of organism, presenting a texture damage degree (TDD) of 0.30.

### Example 8

A ferritic stainless steel material containing C: 0.039 mass%, Si: 0.28 mass%, Mn: 0.26 mass%, P: 0.012 mass%, S: 0.009 mass%, and Cr: 18.3 mass% with the remainder being Fe and unavoidable impurities, was subjected to continuous casting process, followed by hot rolling, cold rolling and bright annealing, and then high-precision worked into a stainless steel needle having an outside diameter of 0.005 mm, an inside diameter of 0. 002 mm and a length of 50 mm. After supersonically cleaned, the needle was coated with an SiNₓ film by means of the magnetron sputtering system shown in Figs.8 and 9 in the high plasma atmosphere.

The magnetron sputtering process formed a 0.5 µm-thick SiNₓ film under conditions of Ar: 100 sccm and N₂: 130 sccm. Thickness measurement was taken the same way as in Example 1.

In this process, the vacuum on the outer surface of the needle was controlled to 1.5×10⁻³ Torr, whereas the vacuum inside of the needle was controlled to 5.0×10⁻⁵ Torr.

A coverage area of the SiNₓ ceramic film on the outer surface was 5 mm from the needle tip, whereas a coverage area of that on the inner surface was 20 µm from the needle tip (aperture bottom).

When the SiNₓ ceramic-coated needle was used to cut off a cell nucleus, a precise cut-off of the nucleus at an intended site could be achieved.

As a matter of course, the needle had substantially no negative effects on the tissues of the organism such as destruction or tear-off, presenting a texture damage degree (TDD) of 0.30.

### Example 9

A ferritic stainless steel material containing C: 0.03 mass%, Si: 0.06 mass%, Mn: 1.3 mass%, P: 0.031 mass%, S: 0.012 mass%, and Cr: 17.9 mass% with the remainder being Fe and unavoidable impurities, was subjected to continuous casting process, followed by hot rolling, cold rolling and bright annealing, and then high-precision worked into metal needles having different outside diameters and inside diameters.

After supersonically cleaned, the needles were coated with various ceramic films by means of the magnetron sputtering system shown in Figs. 8 and 9 in the high plasma atmosphere. The resultant films had a thickness of 1.0 µm. Thickness measurement was taken the same way as in Example 1.

In this process, the metal needles were set the same way as in Fig. 9 while each of various materials listed in Table 6 was used as the sputter target.

Table 6 shows the results of examination on the insulating performance (resistance ρ) and the texture damage degree (TDD) of the ceramic films of the ceramic-coated needles.

As shown in Table 6, the ceramic-coated needles according to the invention had quite excellent insulating performance and presented favorable texture damage degrees of low values.

### Example 10

A stainless steel material containing C: 0.042 mass%, Si: 0.31 mass%, Mn: 0.21 mass%, P: 0.011 mass%, S: 0.008 mass%, and Cr: 17.9 mass% with the remainder being Fe and unavoidable impurities, was subj ected to hot rolling, cold rolling and bright annealing, and then high-precision worked into a stainless steel needle having an outside diameter of 0.005 mm, an inside diameter of 0.002 mm and a length of 50 mm. After supersonically cleaned, the needle was coated with an SiNₓ film by means of the magnetron sputtering system shown in Figs.8 and 9 in the high plasma atmosphere.

The magnetron sputtering process formed a 0.5 µm-thick SiNₓ ceramic film under conditions of Ar: 100 sccm and N₂: 130 sccm. Thickness measurement was taken the same way as in Example 1.

In this process, the vacuum on the outer surface of the needle was controlled to 1.60×10⁻¹ Pa (1.2×10⁻³ Torr), whereas the vacuum inside of the needle was controlled to 9.31×10⁻³ Pa (7.0×10⁻⁵ Torr).

A coverage area of the SiNₓ ceramic film on the outer surface was 50 mm from the needle tip, whereas a coverage area (area represented by 'h' in Fig.10) of that on the inner surface was 50 µm from the needle tip (aperture bottom).

The resultant SiNₓ ceramic-coated needle had a surface roughness Ra of 1.1 µm. When this needle was used to cut off a cell nucleus, a precise cut-off of the nucleus at an intended site could be achieved.

As a matter of course, the needle had substantially no negative effects on the tissues of the organism such as destruction or tear-off, presenting a texture damage degree (TDD) of 0.29.

### Example 11

A stainless steel material containing C: 0.033 mass%, Si: 0.22 mass%, Mn: 0.13 mass%, P: 0.009 mass%, S: 0.012 mass%, and Cr: 18.6 mass% with the remainder being Fe and unavoidable impurities, was subjected to continuous casting process, followed by hot rolling, cold rolling and bright annealing, and then high-precision worked into metal needles having different outside diameters and inside diameters.

After supersonically cleaned, the needles were coated with various ceramic films by means of the magnetron sputtering system shown in Figs.8 and 9 in the high plasma atmosphere, respectively. Thickness measurement was taken the same way as in Example 1.

In this process, the metal needles were set the same way as in Fig. 9 while each of various materials listed in Table 7 was used as the sputter target.

Table 7 shows the results of examination on the surface roughness (Ra) , the insulating performance (resistance ρ) and the texture damage degree (TDD) of the ceramic films of the ceramic-coated needles.

As shown in Table 7, the ceramic-coated needles according to the invention achieved the surface roughness of 1.3 µm or less, also having quite excellent insulating performance and favorable texture damage degrees of low values.

### Example 12

A stainless steel material containing C: 0.023 mass%, Si: 0.26 mass%, Mn: 0.18 mass%, P: 0.01 mass%, S: 0.011 mass%, and Cr: 17.3 mass% with the remainder being Fe and unavoidable impurities, was subjected to continuous casting process, followed by hot rolling, cold rolling and bright annealing, and then high-precision worked into metal needles having different outside diameters and inside diameters.

Similarly to Example 11, the needles were supersonically cleaned and then coated with various ceramic films by means of the magnetron sputtering system shown in Figs.8 and 9 in the high plasma atmosphere, respectively. Thickness measurement was taken the same way as in Example 1.

In the course of the plasma process, O₂ was introduced in the atmosphere to obtain the ceramic films functionally gradient in the thickness direction.

Table 8 shows the results of examination on the surface roughness (Ra), the insulating performance (resistance ρ) and the texture damage degree (TDD) of the ceramic films of the ceramic-coated needles.

As shown in Table 8, the ceramic-coated needles according to the invention achieved the surface roughness of 1.2 µm or less, also having quite excellent insulating performance and favorable texture damage degrees of low values.

### Industrial Applicability

According to the invention, the ceramic-coated medical appliances can be fabricated in a stable manner, the appliances featuring such an excellent insulating performance as to exert no negative effects, such as destruction or tear-off, on the tissues or cells of the organism subjected to surgery or the like.

According to the invention, the ceramic-coated medical needles can be fabricated in a stable manner, the needles having no risk of breakage during use nor negative effects on the sliced tissues or the tissue area around the needle at the site of penetration into the lesion.

According to the invention, the genetic control operation, such as cut-off of the cellular nucleus or injection of the immunological solution or the like into the nucleus, can be performed easily without causing any negative effects on the area around the needle punctured into the nucleus nor on the cellular nucleus in the needle.

**TABLE 1**

| No. | Medical Appliance | Ceramic Type | Resistance ρ (Ω·m) | Adhesion | | Note |
|---|---|---|---|---|---|---|
| | | | | (mm) | Grade | |
| 1 | Biopsy forceps | SiNₓ | ∞ | 20 | ○ | Example |
| 2 | Forceps | SiO₂ | ∞ | 25 | Δ | Example |
| 3 | Surgical scissors | BN | ∞ | 25 | Δ | Example |
| 4 | Surgical knife | Al₂O₃ | ∞ | 30 | Δ | Example |
| 5 | Biopsy forceps | TiN | 2.0×10⁴ | 20 | ○ | Comparative Example |
| ∞: resistance ρ≥10⁹ Ω·m | | | | | | |

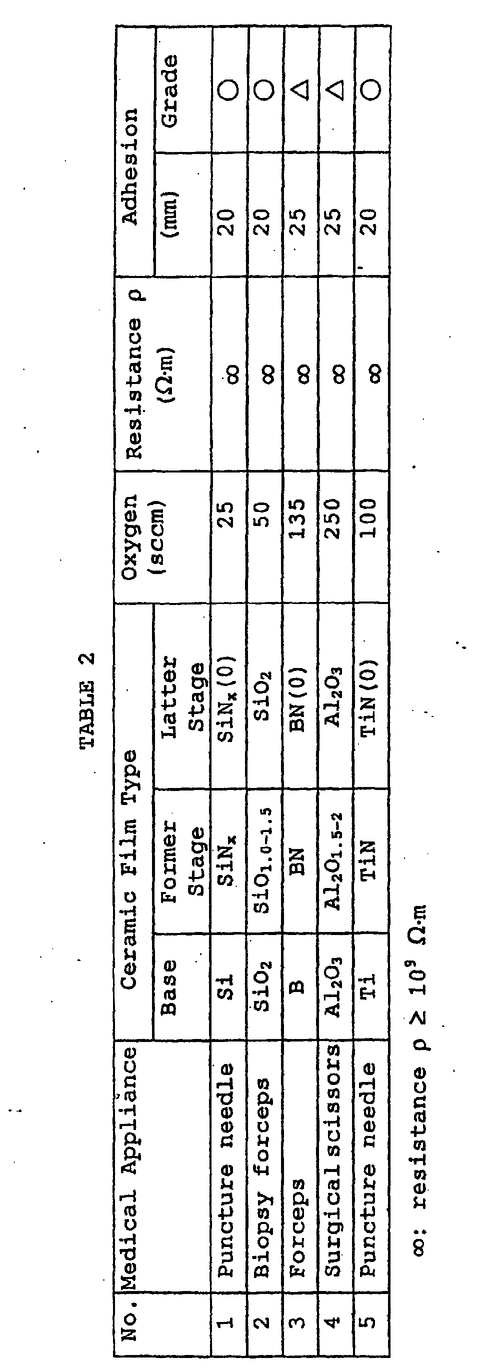

**TABLE 3**

| Item | Resistance ρ (Ω·m) | Adhesion | | TDD | Note |
|---|---|---|---|---|---|
| | | (mm) | Grade | | |
| SiNₓ formed on inner&outer surfaces of (a) | ∞ | 20 | ○ | 0.10 | Example |
| SiNₓ formed on inner&outer surfaces of (b) | ∞ | 30 | Δ | 0.15 | Example |
| (a) free from film | 7.0×10⁻⁶ | - | - | 0.5 | Comparative Example |
| ∞: resistance ρ≥10⁹ Ω·m | | | | | |

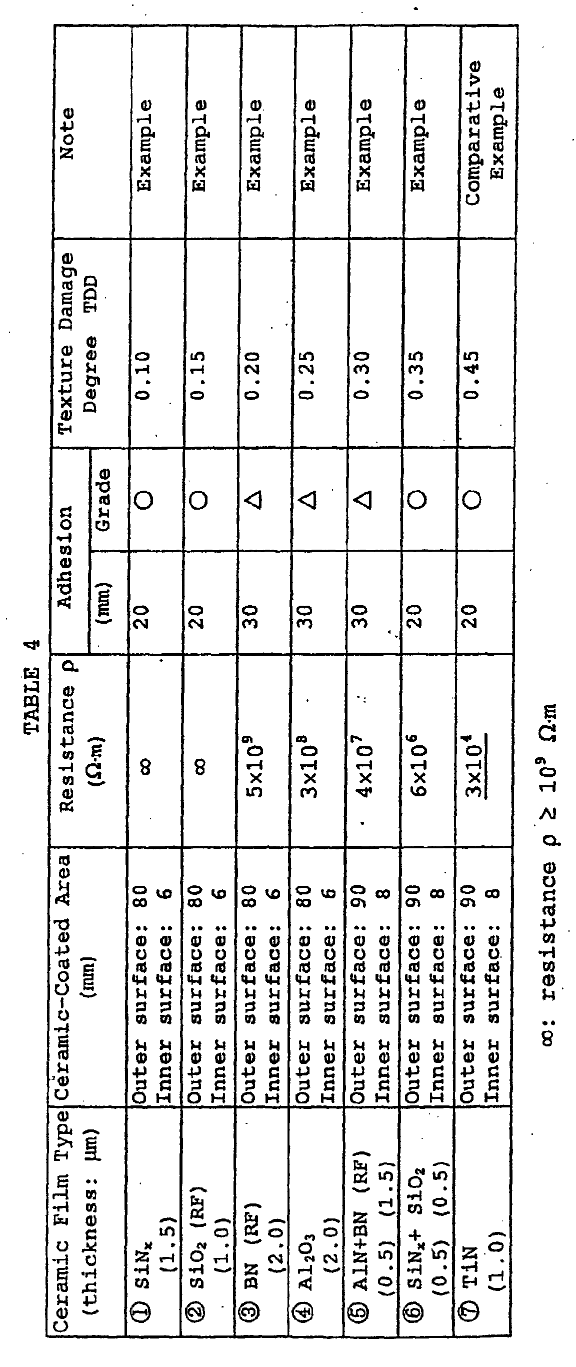

**TABLE 5**

| No. | Target Type | Cathode Type | Resistance ρ (Ω·m) | Adhesion | | Note |
|---|---|---|---|---|---|---|
| | | | | (mm) | Grade | |
| 1 | Sintered SiO₂ powder | S cathode | ∞ | 20 | ○ | Example |
| 2 | Sintered SiN₄ powder | W cathodes | ∞ | 20 | ○ | Example |
| 3 | Sintered BN powder | S cathode | ∞ | 25 | Δ | Example |
| 4 | Sintered Al₂O₃ powder | W cathodes | ∞ | 25 | Δ | Example |
| 5 | Sintered TiN powder | S cathode | 2.0×10⁴ | 20 | ○ | Comparative Example |
| S cathode: single cathode system, W cathodes: W cathode system, ∞: resistance ρ≥10⁹ Ω·m | | | | | | |

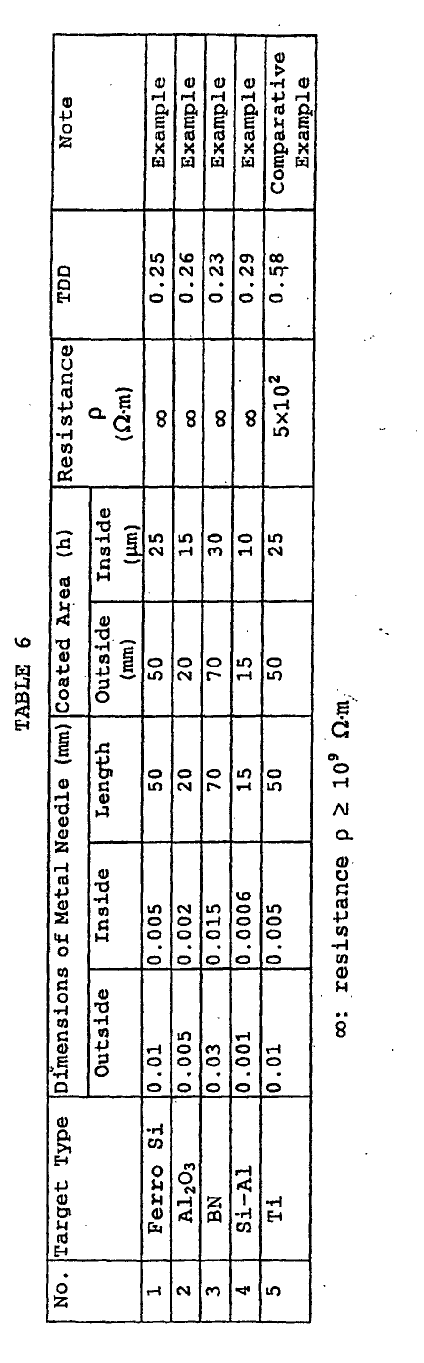

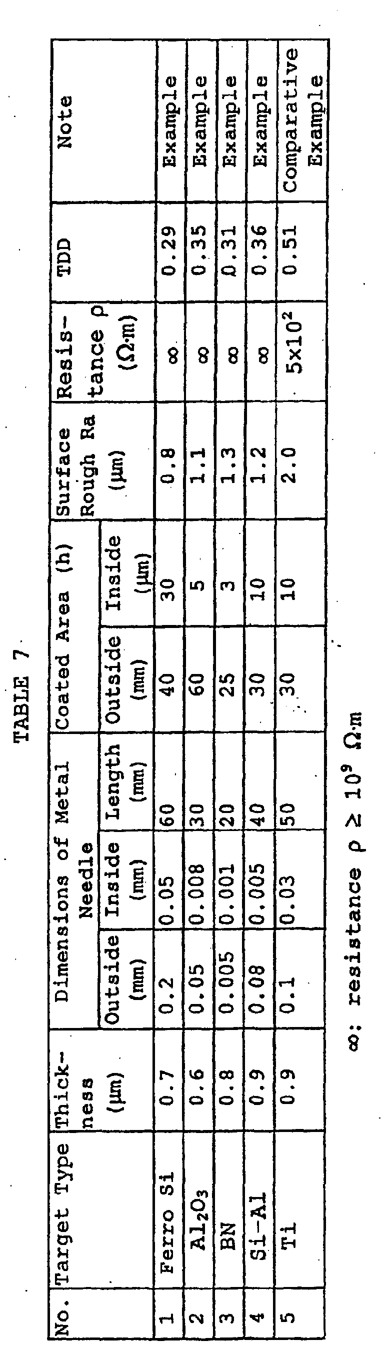

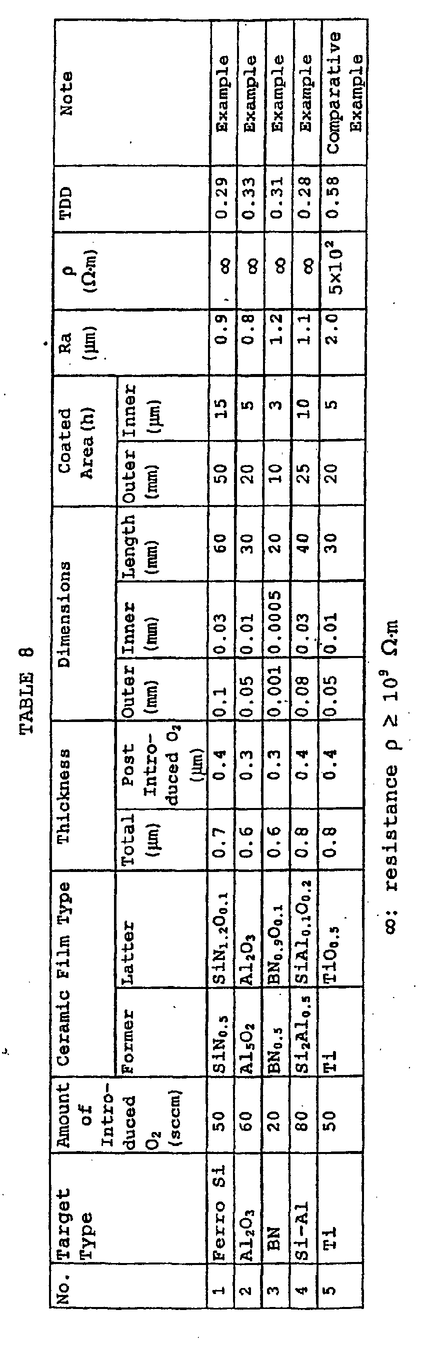

## Claims

1. A ceramic-coated medical appliance or biopsy appliance wherein at least a metal potion directly contacting an organism is coated with an insulating ceramic film having a resistance ρ of at least 10⁵ Ω·m.

2. A ceramic-coated medical appliance or biopsy appliance as claimed in Claim 1, wherein the ceramic film has a thickness of 0.05 to 5.0 µm.

3. A ceramic-coated medical appliance or biopsy appliance as claimed in Claim 1 or 2, wherein the ceramic film comprises at least one selected from the group consisting of nitrides, carbides or oxides of Al, B, Si, Cr and Ti.

4. A ceramic-coated medical appliance as claimed in any one of Claims 1 to 3, wherein the medical appliance is any one of biopsy forceps, forceps, surgical scissors and a surgical knife.

5. A ceramic-coated medical appliance as claimed in any one of Claims 1 to 3, **characterized in that** the ceramic-coatedmedical appliance is any one of an injection needle, puncture needle and suture needle.

6. A ceramic-coated medical needle as claimed in Claim 5, **characterized in that** the insulating ceramic film of a resistance ρ of 10⁵ Ω·m or more is formed on a part or the whole of a surface of the needle.

7. A ceramic-coated medical needle as claimed in Claim 6, wherein the ceramic film has a thickness of 0.05 to 5.0 µm.

8. A ceramic-coated medical needle as claimed in Claim 6 or 7, wherein the ceramic film comprises at least one selected from the group consisting of nitrides, carbides or oxides of Al, B, Si, Cr and Ti.

9. A ceramic-coated medical needle as claimed in any one of Claims 6 ,7 or 8, wherein a substrate metal of the coated needle is stainless steel or high tension steel.

10. A ceramic-coated medical needle as claimed in Claim 6, **characterized in that** a ceramic-coated area of the needle is a part or the whole of an outer surface thereof and a portion of an inner surface thereof that is 1 mm inward from a tip of the needle.

11. A ceramic-coated medical needle as claimed in Claim 10, wherein the ceramic film has a thickness of 0.05 to 5.0 µm.

12. A ceramic-coated medical needle as claimed in Claim 10 or 11, wherein the ceramic film comprises at least one selected from the group consisting of nitrides, carbides or oxides of Al, B, Si, Cr and Ti.

13. A ceramic-coated medical needle as claimed in any one of Claims 10, 11 or 12, wherein a substrate metal of the coated needle is stainless steel or high tension steel.

14. A ceramic-coated genetic control needle as claimed in any one of Claims 1 to 3, wherein the biopsy appliance is a genetic control needle.

15. A ceramic-coated genetic control needle as claimed in Claim 14, wherein a ceramic-coated area of the metal needle is at least a portion of a surface thereof that contacts a cell nucleus in tissue of an organism.

16. A ceramic-coated genetic control needle as claimed in claim 14 or 15, wherein the coated needle has a diameter of 0.0005 to 0.5 mm.

17. A ceramic-coated genetic control needle as claimed in any one of Claims 14, 15 or 16, wherein the ceramic film comprises at least one selected from the group consisting of nitrides, oxides or carbides of Al, B, Si, Cr and Ti.

18. A ceramic-coated genetic control needle as claimed in Claim 14, **characterized in that** a ceramic-coated area of an outer surface of the metal needle is at least a portion thereof that contacts tissue of an organism, whereas a ceramic-coated area of an inner surface of the needle is at least a portion thereof that contacts a cell nucleus in the tissue of the organism.

19. A ceramic-coated genetic control needle as claimed in Claim 18, **characterized in that** the coverage of the insulating ceramic film on the inner surface of the metal needle extends 10 µm or more from a tip. of the metal needle.

20. A ceramic-coated genetic control needle as claimed in Claim 18 or 19, wherein the ceramic film has a thickness of 0.05 to 5.0 µm.

21. A ceramic-coated genetic control needle as claimed in any one of Claims 18, 19 or 20, wherein the ceramic film comprises at least one selected from the group consisting of nitrides, carbides or oxides of Al, B, Si, Cr and Ti.

22. A ceramic-coated genetic control needle as claimed in any one of Claims 18 to 21, wherein a substrate metal of the needle is stainless steel or high tension steel.

23. A ceramic-coated genetic control needle as claimed in any one of Claims 18 to 22, wherein the needle has a diameter φ of 0.0005 to 0.5 mm.

24. A ceramic-coated genetic control needle as claimed in Claim 14, **characterized in that** a ceramic-coated area of the metal needle is at least a respective portion of an outer and an inner surface thereof that contacts a cell nucleus in tissue of an organism, and that the ceramic film has an arithmetic mean surface roughness Ra of 1.5 µm or less.

25. A ceramic-coated genetic control needle as claimed in Claim 24, wherein the coverage of the insulating ceramic film on the inner surface extends at least 10 µm from a tip of the needle.

26. A ceramic-coated genetic control needle as claimed in Claim 24 or 25, wherein the ceramic film has a thickness of 0.05 to 5.0 µm.

27. A ceramic-coated genetic control needle as claimed in any one of Claims 24, 25 or 26, wherein the ceramic film comprises at least one selected from the group consisting of nitrides, carbides or oxides of Al, B, Si, Cr and Ti.

28. A ceramic-coated genetic control needle as claimed in any one of Claims 24 to 27, wherein a substrate metal of the needle is stainless steel or high tension steel.

29. A ceramic-coated genetic control needle as claimed in any one of Claims 24 to 28, wherein the needle has a diameter φ of 0.0005 to 0.5 mm.

30. A fabrication method for ceramic-coated medical appliance or biopsy appliance wherein an insulating ceramic coating film is formed by dry plating at least on a metal portion of a metallic medical appliance or biopsy appliance that directly contacts an organism.

31. A fabrication method for ceramic-coated medical appliance or biopsy appliance as claimed in Claim 30, **characterized in that** 5 to 500 scccm of O₂ is introduced in a coating atmosphere in the latter stage of the dry plating process, thereby offering a ceramic-coated medial appliance or biopsy appliance having excellent insulating performance, adhesion and wear resistance and featuring a resistance ρ of 10⁵ Ω·m or more at least at a top surface of the ceramic film.

32. A fabrication method for ceramic-coated medical appliance or biopsy appliance as claimed in Claim 30 or 31, wherein the top surface of the ceramic film has a resistance ρ of 10⁹ Ω·m or more.

33. A fabrication method for ceramic-coated medical appliance or biopsy appliance as claimed in any one of Claims 30, 31 or 32, **characterized in that** the ceramic film comprises at least one selected from the group consisting of nitrides, oxides or carbides of Al, B, Si, Cr and Ti.

34. A fabrication method for ceramic-coated medical appliance as claimed in any one of Claims 30 to 33, **characterized in that** the medical appliance is anyone of biopsy forceps , forceps, surgical scissors and a surgical knife.

35. A fabrication method for ceramic-coated medical appliance as claimed in any one of Claims 30 to 33, **characterized in that** the medical appliance is an inj ection needle or puncture needle.

36. A fabrication method for ceramic-coated medical needle as claimed in Claim 35, wherein the metal needle is set in parallel with an approaching direction of deposition particles as presenting a tip thereof against the approaching deposition particles and the ceramic film is formed at least on the tip portion of the needle by dry plating.

37. A fabrication method for ceramic-coated medical needle as claimed in Claim 36, wherein the ceramic film is an insulating ceramic film having a resistance ρ of 10⁵ Ω·m or more.

38. A fabrication method for ceramic-coated medical needle as claimed in Claim 36 or 37, wherein the ceramic film has a thickness of 0.05 to 5.0 µm.

39. A fabrication method for ceramic-coated medical needle as claimed in Claim 36, 37 or 38, wherein the ceramic film comprises at least one selected from the group consisting of nitrides, carbides or oxides of Al, B, Si, Cr and Ti.

40. A fabrication method for ceramic-coated medical needle as claimed in any one of Claims 36 to 39, wherein a substrate metal of the coated needle is stainless steel or high tension steel.

41. A fabrication method for ceramic-coated medical needle as claimed in Claim 35, wherein the insulating ceramic film is formed by magnetron sputtering process using, as a sputter target, at least one selected from the group consisting of nitrides, carbides or oxides of Al, B, Si, Cr and Ti while preventing abnormal discharge by disposing a high-plasma atmosphere forming magnet and an RF device around the target, thereby overlaying the cerami c film on a part or the whole of an outer surface of the needle and on a portion of an inner surface thereof at least 1 mm inward from a tip of the needle.

42. A fabrication method for ceramic-coated medical needle as claimed in Claim 41, wherein two of the sputter targets are disposed for forming the insulating ceramic film based on a W-cathode system.

43. A fabrication method for ceramic-coated medical needle as claimed in Claim 41 or 42, **characterized in that** the metal needle is set in parallel with an approaching direction of deposition particles as presenting its tip against the approaching deposition particles.

44. A fabrication method for ceramic-coated medical needle as claimed in any one of Claims 41, 42 or 43, wherein the supply of reaction gas is suspended in an initial stage of the magnetron sputtering process.

45. A fabrication method for ceramic-coated genetic control needle appliance as claimed in any one of Claims 30 to 33, wherein the biopsy appliance is a genetic control needle.

46. A fabrication method for ceramic-coated genetic control needle as claimed in Claim 45, wherein the metal needle is set in parallel with an approaching direction of depositionparticles as presenting a tip thereof against the approaching deposition particles, and wherein the insulating ceramic film having a resistance ρ of 10⁵ Ω·m or more is formed at least on the tip portion of the needle by dry plating with an interior of the needle maintained at higher vacuum than the outside thereof through differential-pressure evacuation.

47. A system for fabricating a ceramic-coated genetic control needle comprising a magnetron sputtering apparatus, a sample holder and a differential-pressure high-vacuum chamber in a vacuum vessel of a dry plating system, wherein a metal needle as a coating object is set on the sample holder as positioned in parallel with an approaching direction of depositionparticles and presenting a tip thereof against the approaching deposition particles, and wherein a trailing end of the needle is connected with the differential-pressure high-vacuum chamber via a tube for maintaining an interior of the metal needle at higher vacuum than outside there of by differential pressure vacuum effect while an insulating ceramic film is formed on an outer and an inner surface of the metal needle.
